# EUROPEAN PATENT SPECIFICATION

(11) **EP 1 129 112 B1**
(45) Date of publication and mention of the grant of the patent: **25.10.2006**
(21) Application number: 99912635.2
(22) Date of filing: 12.03.1999
(51) Int. Cl.: C07K 14/705, C12N 15/82, G01N 33/68, C12N 15/11

(54) **PEPTIDES CONTAINING A CHOLESTEROL RECOGNITION SEQUENCE AND THEIR USES**
PEPTIDE ENTHALTEND EINE CHOLESTERIN-ERKENNUNGSSEQUENZ UND DEREN VERWENDUNGEN
SEQUENCE DE RECONNAISSANCE DU CHOLESTEROL

(30) Priority: 12.03.1998 US 77753 P
(43) Date of publication of application: 05.09.2001
(60) Divisional application: 06076592.2
(73) Proprietor: Georgetown University, Washington, DC 20057 (US)
(72) Inventor: PAPADOPOULOS, Vassilios, North Potomac, MD 20878 (US); LI, Hua, Arlington, VA 22209 (US)
(74) Representative: Daniels, Jeffrey Nicholas
(86) International application number: PCT/US1999/005853
(87) International publication number: WO 1999/046575

(56) References cited:
- WO-A1-95/03325
- WO-A1-97/18826
- WO-A1-99/46389
- J R GLENNEY JR. : "The sequence of human caveolin reveals identity with VIP21, a component of transport vesicles" FEBS LETTERS., vol. 314, no. 1, 1992, pages 45-48, XP002154826 ELSEVIER SCIENCE PUBLISHERS, AMSTERDAM., NL ISSN: 0014-5793
- T P BOYLE & K R MAROTTI: "Structure of the murine gene encoding apolipoprotein A-1" GENE., vol. 117, 1992, pages 243-247, XP002154827 ELSEVIER BIOMEDICAL PRESS. AMSTERDAM., NL ISSN: 0378-1119 cited in the application
- V PAPADOPOULOS ET AL.: "Targeted disruption of the peripheral-type benzodiazepine receptor gene inhibits steroidogenesis in the R2C Lexdig tumor cell line" JOURNAL OF BIOLOGICAL CHEMISTRY., vol. 22, no. 51, 19 December 1997 (1997-12-19), pages 32129-22135, XP002114579 AMERICAN SOCIETY OF BIOLOGICAL CHEMISTS, BALTIMORE, MD., US ISSN: 0021-9258
- DATABASE MEDLINE [Online] US NATIONAL LIBRARY OF MEDICINE (NLM), BETHESDA, MD, US; PIKULEVA I A ET AL: "Active-site topology of bovine cholesterol side-chain cleavage cytochrome P450 (P450scc) and evidence for interaction of tyrosine 94 with the side chain of cholesterol." retrieved from STN Database accession no. 96004795 XP002154829 & ARCHIVES OF BIOCHEMISTRY AND BIOPHYSICS, (1995 SEP 10) 322 (1) 189-97. ,
- DATABASE MEDLINE [Online] US NATIONAL LIBRARY OF MEDICINE (NLM), BETHESDA, MD, US; SU P ET AL: "A cDNA encoding a rat mitochondrial cytochrome P450 catalyzing both the 26-hydroxylation of cholesterol and 25-hydroxylation of vitamin D3: gonadotropic regulation of the cognate mRNA in ovaries." retrieved from STN Database accession no. 91083838 XP002154830 & DNA AND CELL BIOLOGY, (1990 NOV) 9 (9) 657-67. ,
- DATABASE CHEMABS [Online] CHEMICAL ABSTRACTS SERVICE, COLUMBUS, OHIO, US; FARGES, ROSELI ET AL: "Site-directed mutagenesis of the peripheral benzodiazepine receptor: identification of amino acids implicated in the binding site of Ro5-4864" retrieved from STN Database accession no. 122:122952 CA XP002154831 & MOL. PHARMACOL. (1994), 46(6), 1160-7 , 1994,
- H LI & V PAPADOPOULOS: "Peripheral-type benzodiazepine receptor function in cholesterol transport. Identification of a putative cholesterol recognition/interaction amino acid sequence and consensus pattern" ENDOCRINOLOGY., vol. 139, no. 12, 1999, pages 4991-4997, XP002154828 BALTIMORE, MD., US ISSN: 0013-7227
- M HARDWICK ET AL.: "Peripheral-type benzodiazepine receptor (PBR) in human breast cancer: correlation of bbreast cancer cell aggressive phenotype with PBR expression, nuclear localization, and PBR-mediated cell proliferation and nuclear transport of cholesterol " CANCER RESEARCH., vol. 59, 15 February 1999 (1999-02-15), pages 831-842, XP002114583 AMERICAN ASSOCIATION FOR CANCER RESEARCH, BALTIMORE, MD., US ISSN: 0008-5472
- V PAPADOPOULOS : "Structure and function of the peripheral-type benzodiazepine receptor in steroidogenic cells" PROCEEDINGS OF THE SOCIETY FOR EXPERIMENTAL BIOLOGY & MEDICINE., vol. 217, no. 2, February 1998 (1998-02), pages 130-142, XP002154897 ACADEMIC PRESS INC. NEW YORK., US ISSN: 0037-9727
- K E KRUEGER & V PAPADOPOULOS: "Peripheral-type benzodiazepine receptors mediate translocation from outer to inner mitochondrial membranes in adrenocortical cells" JOURNAL OF BIOLOGICAL CHEMISTRY., vol. 65, no. 25, 5 September 1990 (1990-09-05), pages 15015-15022, XP002133901 AMERICAN SOCIETY OF BIOLOGICAL CHEMISTS, BALTIMORE, MD., US ISSN: 0021-9258
- H MIETTINEN ET AL.: "Expression of peripheral-type benzodiazepine receptor and diazepam binding inhibitor in human astrocytomas; relationship to cell proliferation" CANCER RESEARCH., vol. 55, 15 June 1995 (1995-06-15), pages 2691-2695, XP002114581 AMERICAN ASSOCIATION FOR CANCER RESEARCH, BALTIMORE, MD., US ISSN: 0008-5472
- JOURNAL OF BIOLOGICAL CHEMISTRY, vol. 269, no. 10, 1994, pages 7527-7531, US AMERICAN SOCIETY OF BIOLOGICAL CHEMISTS, BALTIMORE, MD.

### SEQUENCE LISTING

<110> GEORGETOWN UNIVERSITY MEDICAL CENTER
<120> CHOLESTEROL RECOGNITION SEQUENCE
<130> 23521-0118
<140> PCT/US99/05853
   <141> 1999-03-12
<160> 25
<170> PatentIn Ver. 2.1
<210> 1
   <211> 7
   <212> PRT
   <213> Artificial Sequence
<220>
   <223> Description of Artificial Sequence: synthetic peptide
<400> 1
   Leu Asn Tyr Tyr Val Trp Arg
    1               5
<210> 2
   <211> 7
   <212> PRT
   <213> Artificial Sequence
<220>
   <223> Description of Artificial Sequence: synthetic peptide
<400> 2
   Leu Asn Tyr Cys Val Trp Arg
    1               5
<210> 3
   <211> 4
   <212> PRT
   <213> Artificial Sequence
<220>
   <223> Description of Artificial Sequence: synthetic peptide
<400> 3
   Leu Asn Tyr Arg
   1
<210> 4
   <211> 9
   <212> PRT
   <213> Artificial Sequence
<220>
   <223> Description of Artificial Sequence: synthetic peptide
<400> 4
   Val Ala Tyr His Gln Tyr Tyr Gln Arg
   1 5
<210> 5
   <211> 8
   <212> PRT
   <213> Artificial Sequence
<220>
   <223> Description of Artificial Sequence: synthetic peptide
<400> 5
   Val Leu Asn Tyr Tyr Val Trp Arg
   1 5
<210> 6
   <211> 7
   <212> PRT
   <213> Artificial Sequence
<220>
   <223> Description of Artificial Sequence: synthetic peptide
<400> 6
   Leu Asn Tyr Tyr Val Trp Arg
   1 5
<210> 7
   <211> 7
   <212> PRT
   <213> Artificial Sequence
<220>
   <223> Description of Artificial Sequence: synthetic peptide
<400> 7
   Leu Asn Tyr Cys Val Trp Arg
   1 5
<210> 8
   <211> 4
<212> PRT
   <213> Artificial Sequence
<220>
   <223> Description of Artificial Sequence: synthetic peptide
<400> 8
   Leu Asn Tyr Arg
   1
<210> 9
   <211> 9
   <212> PRT
   <213> Artificial Sequence
<220>
   <223> Description of Artificial Sequence: synthetic peptide
<400> 9
   Val Ala Tyr His Gln Tyr Tyr Gln Arg
   1 5
<210> 10
   <211> 9
   <212> PRT
   <213> Artificial Sequence
<220>
   <223> Description of Artificial Sequence: synthetic peptide
<400> 10
   Val Ala Tyr His Gln Tyr Tyr Gln Arg
   1 5
<210> 11
   <211> 9
   <212> PRT
   <213> Artificial Sequence
<220>
   <223> Description of Artificial Sequence: synthetic peptide
<400> 11
   Val Ala Tyr His Glx His Tyr Gln Lys
   1 5
<210> 12
   <211> 7
   <212> PRT
   <213> Artificial Sequence
<220>
   <223> Description of Artificial Sequence: synthetic peptide
<400> 12
   Leu Asn Glu Tyr His Thr Arg
   1 5
<210> 13
   <211> 8
   <212> PRT
   <213> Artificial Sequence
<220>
   <223> Description of Artificial Sequence: synthetic peptide
<400> 13
   Val Thr Lys Tyr Trp Phe Tyr Arg
   1 5
<210> 14
   <211> 8
   <212> PRT
   <213> Artificial Sequence
<220>
   <223> Description of Artificial Sequence: synthetic peptide
<400> 14
   Val Phe Tyr Val Ile Glu Thr Arg
   1 5
<210> 15
   <211> 8
   <212> PRT
   <213> Artificial Sequence
<220>
   <223> Description of Artificial Sequence: synthetic peptide
<400> 15
   Leu Phe Ile Tyr Ser His Phe Lys
   1 5
<210> 16
   <211> 11
   <212> PRT
   <213> Artificial Sequence
<220>
   <223> Description of Artificial Sequence: synthetic peptide
<400> 16
   Leu Cys Ala Gly Ser Ser Tyr Arg His Met Arg
   1 5 10
<210> 17
   <211> 12
   <212> PRT
   <213> Artificial Sequence
<220>
   <223> Description of Artificial Sequence: synthetic peptide
<400> 17
   Val Tyr Lys Glu Met Tyr Lys Thr Asp Leu Glu Lys
   1 5 10
<210> 18
   <211> 9
   <212> PRT
   <213> Artificial Sequence
<220>
   <223> Description of Artificial Sequence: synthetic peptide
<400> 18
   Val Leu Cys Thr Tyr Val Val Ser Arg
   1 5
<210> 19
   <211> 9
   <212> PRT
   <213> Artificial Sequence
<220>
   <223> Description of Artificial Sequence: synthetic peptide
<400> 19
   Val Ser Leu Tyr Leu Ala Ile Thr Lys
   1 5
<210> 20
   <211> 11
   <212> PRT
   <213> Artificial Sequence
<220>
   <223> Description of Artificial Sequence: synthetic peptide
<400> 20
   Val Thr Glu Gly Met Tyr Ala Phe Cys Tyr Arg
   1 5 10
<210> 21
   <211> 8
   <212> PRT
   <213> Artificial Sequence
<220>
   <223> Description of Artificial Sequence: synthetic peptide
<400> 21
   Val Thr Glu Ala Tyr Arg Gln Arg
   1 5
<210> 22
   <211> 10
   <212> PRT
   <213> Artificial Sequence
<220>
   <223> Description of Artificial Sequence: synthetic peptide
<400> 22
   Leu Ser Pro Tyr Asn Lys Gly Leu Ile Arg
   1 5 10
<210> 23
   <211> 9
   <212> PRT
   <213> Artificial Sequence
<220>
   <223> Description of Artificial Sequence: synthetic peptide
<400> 23
   Val Val Asp Tyr Ile Asp Glu Gly Arg
    1               5
<210> 24
   <211> 28
   <212> DNA
   <213> Artificial Sequence
<220>
   <223> Description of Artificial Sequence: primer
<400> 24
   atatatacat atgcctgaat cctgggtg 28
<210> 25
   <211> 26
   <212> DNA
   <213> Artificial Sequence
<220>
   <223> Description of Artificial Sequence: primer
<400> 25
   atactcgagt gggtgccttc actctg 26

## Description

### INTRODUCTION

The primary point of control in the acute stimulation of steroidogenesis by hormones involves the first step in this biosynthetic pathway where cholesterol is converted to pregnenolone by the C₂₇ cholesterol side chain cleavage cytochrome P-450 enzyme (p-450scc) and auxiliary electron transferring proteins, localized on inner mitochondrial membranes (IMM) (Simpson, E.R. and Waterman, M. R., 1983, *Can. J. Biochem. Cell. Biol.* 61:692-717; Jefcoate, C. R. *et al.* 1992, *J. Steroid Biochem. Molec. Biol.* 43: 751-767). More detailed studies have shown that the reaction catalyzed by P-450scc is not the rate-limiting step in the synthesis of steroid hormones. Rather, the rate-limiting step is the transport of the precursor, cholesterol, from intracellular sources to the IMM. This hormone-dependent transport mechanism was shown to be localized in the mitochondrion (Simpson and Waterman, 1983, *supra;* Jefcoate *et al.,* 1992, *supra).* All documents cited herein *supra* and *infra* are hereby incorporated in their entirety by reference thereto.

The peripheral-type benzodiazepine receptor (PBR) was originally discovered because it binds the benzodiazepine diazepam with relatively high affinity (Papadopoulos, V. 1993, *Endocr. Rev.* 14:222-240). Benzodiazepines are among the most highly prescribed drugs due to their pharmacological actions in relieving anxiety mediated through modulating the activity of γ-aminobutyric acid receptors in the central nervous system (Costa, E. and Guidotti, A. 1979, *Annu. Rev. Pharmacol. Toxicol.* 19:531-545). PBR is another class of binding sites for benzodiazepines distinct from the aforementioned neurotransmitter receptors. Further studies demonstrated that in addition to benzodiazepines, PBR binds other classes of organic compounds with high affinity (Papadopoulos, 1993, *supra).* PBR, although present in all tissues examined, was found to be particularly high in steroid producing tissues, where it was primarily localized in the outer mitochondrial membrane (OMM) (Anholt, R.R.H. *et al.* 1986, *J. Biol. Chem.* 261: 576-583). An 18 kDa isoquinoline-binding protein was identified as PBR, cloned and expressed (Papadopoulos, V. 1998, *Proc Soc. Exp. Biol. Med.* 217:130-142). It was then demonstrated that PBR is a functional component of the steroidogenic machinery (Papadopoulos, 1998, *supra;* Papadopoulos V. *et al.* 1990, *J. Biol. Chem.* 265:3772-3779) mediating cholesterol delivery from the outer to the inner mitochondrial membrane (Krueger, K. E. and Papadopoulos, V. 1990, *J. Biol. Chem.* 265:15015-15022). Further studies demonstrated that pharmacologically induced reduction of adrenal PBR levels *in vivo* resulted in decreased circulating glucocorticoid levels (Papadopoulos, V. 1998, *supra).* In addition, targeted disruption of the PBR gene in Leydig cells resulted in the arrest of cholesterol transport into mitochondria and steroid formation; transfection of the mutant cells with a PBR cDNA rescued steroidogenesis (Papadopoulos, V. *et al.* 1997, *J. Biol. Chem.* 272:32129-32135). How PBR affected cholesterol transport and whether or not PBR directly interacted with cholesterol was not known.

Glenny J.R. (FEBS Letters 314(1), pages 45-49) discloses the cDNA sequence of human lung caveolin. This is homologous to the sequence of chicken caveolin cDNA and VIP 21.

Boyle T.P. and Marotti K.R. (Gene 117, pages 243-247) disclose the sequence of the murine apolipoprotein A-I gene.

Su P. *et a.l.* (Medline Abstract 91083838) discloses screening a cDNA expression library derived from rat liver with a polyclonal antibody specific for mitochondrial vitamin D3 25-hydroxylase and a cDNA for rabbit liver mitochondrial cytochrome P450c26. A bifunctional mitochondrial cytochrome P450 capable of catalysing the 25-hydroxylation of vitamin D3 and the 26-hydroxylation of cholesterol was identified.

Pikuleva I.A. *et al.* (Medline Abstract 96004795) discloses the effect of mutating specific residues of bovine cholesterol side chain cleavage cytochrome P450scc on substrate binding. Pikuleva *et al.* suggests that Y94 iteracts with the side chain of cholesterol.

### SUMMARY OF THE INVENTION

Based on the known amino acid sequence of the human and mouse 18 kDa PBR protein, a three dimensional model of this receptor protein was developed using molecular dynamics simulations (Bernassau, J. M. *et al.* 1993, *J. Mol. Graph.* 11:236-245; Papadopoulos, V. 1996, In: Payne A. H., Hardy, M. P., Russell, L. D. (eds) The Leydig Cell. Cashe River Press, Vienna, IL, pp 598-628). Analysis of the three dimensional structure indicated that both the human' and mouse PBR could possibly accomodate cholesterol and function as a channel. In this invention is described the testing and proof of this hypothesis.

In the present invention, we identify a cholesterol recognition amino acid sequence on PBR, common to all proteins shown to interact with cholesterol, and demonstrate that PBR functions as a cholesterol channel-like structure, mediating cholesterol transport across membranes.

Cholesterol, coming from various intracellular sources, is recognized by the cholesterol recognition/interaction amino acid consensus pattern present in the carboxy-terminal of PBR in the outer mitochondrial membrane (QMM). This pool of cholesterol enters in the (OMM) at the PBR sites where it remains without mixing with other membrane components. Ligand binding to the receptor induces the release of this cholesterol. When it is released, the cholesterol can be accessed by the P450scc and cleaved into pregnenolone, precursor of all steroids.

Therefore, it is an object of the present invention to provide peptides having a cholesterol recognition/interaction amino acid sequence.

The presence of the cholesterol recognition/interaction amino acid sequence in a protein infers the likelihood of interaction with cholesterol.

It is another object of the present invention to provide a nucleotide sequence encoding the cholesterol recognition/interaction amino acid sequence described above, and vectors incorporating all or part of said sequence, and cells, prokaryotic and eukaryotic, transformed or transfected with said vectors, for use in the production of peptides having a cholesterol interaction/recognition sequence. The transformed or transfected cells are useful for screening agents or drugs which alter cholesterol interaction/recognition, and uptake of cholesterol.

It is yet another object of the present invention to provide a method for identifying whether or not a protein recognizes or interacts with cholesterol said method comprising identifying the presence or absence of the above-mentioned cholesterol recognition/interaction amino acid sequence wherein the presence of the sequence is an indication of the likelihood that the protein recognizes/interacts with cholesterol.

It is yet another object of the present invention to provide a method for conferring to a molecule the ability to interact with cholesterol by introducing into a molecule or polypeptide, natural or synthetic, a cholesterol recognition/interaction sequence such that the protein or polypeptide, natural or synthetic, is now able to recognize/interact with cholesterol.

It is still another object of the present invention to provide a molecule, natural or synthetic comprising the cholesterol recognition/interaction sequence described above.

It is a further object of the present invention to provide a molecule which blocks or competes with the cholesterol-interaction/recognition ability of the cholesterol recognition/interaction amino acid sequence described above. The molecule can be an antibody, a peptide, a peptide comprising the cholesterol interaction/recognition consensus sequence, or drug.

It is yet another object of the present invention to provide a method for altering the cholesterol binding ability of molecules which contain the cholesterol recognition/interaction amino acid sequence described above, comprising altering said site such that cholesterol recognition is reduced, eliminated or increased. Cholesterol recognition can be increased by providing a perfect cholesterol pocket, or alternatively, by providing more than one cholesterol recognition/interaction amino acid sequence in a protein molecule.

It is further another object of the present invention to provide a PBR with reduced ability to recognize and interact with cholesterol by modifying the cholesterol recognition/interaction site of PBR, for example by changing amino acid 153 from tyrosine to serine, or by changing amino acid 155 from arginine to leucine. These modification or similar modifications may be used for reducing the cholesterol recognition/interaction or binding of other proteins or agents which contain the cholesterol interaction/recognition ammo acid sequence described above.

### BRIEF DESCRIPTION OF THE DRAWINGS

These and other features, aspects, and advantages of the present invention will become better understood with reference to.the following description and appended claims, and accompanying drawings where:

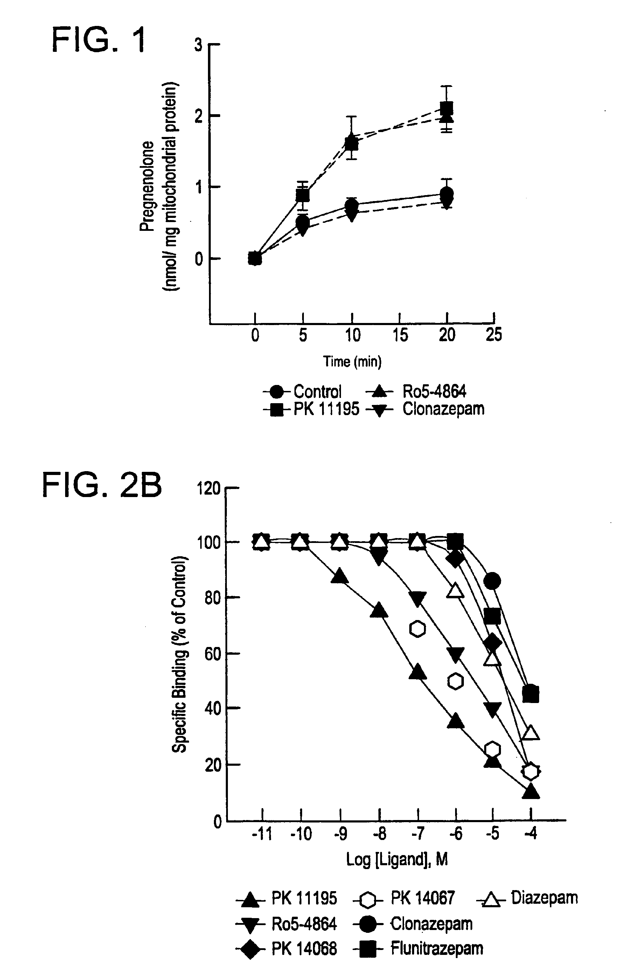

Figure 1 shows effect of PBR ligands on MA-10 Leydig cell cholesterol transport and pregnenolone formation. Mitochondria from MA-10 Leydig cells were incubated with ³H-cholesterol in the presence or absence of the indicated compounds. ³H-Pregnenolone formed was measured as described in Materials and Methods. Data (means ± SD) shown are representative of two to four independent experiments, each having triplicate assays. The effect seen was statisticalyy significant at all times (P<0.001).

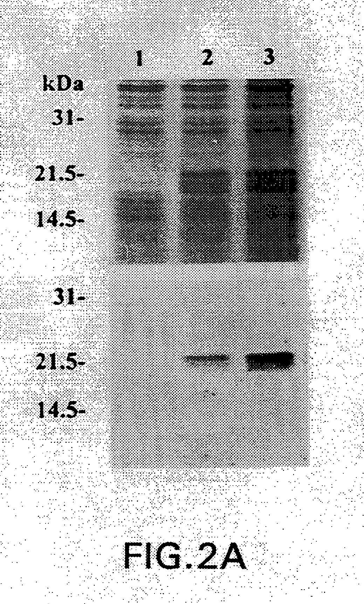

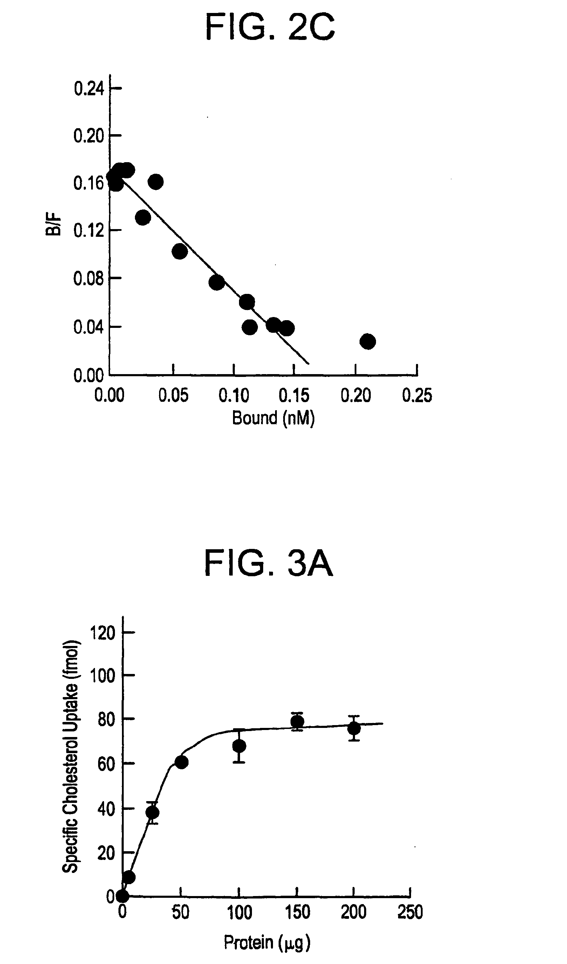

Figure.2 A, B, and C show the expression of recombinant PBR in bacteria. Recombinant mPBR expression vector was developed and used to transform the BL21(DE3) *Escherichia coli* strain as described in Materials and Methods. The expression of recombinant mPBR protein was induced by 1 mM IPTG.
   A) PBR protein expression was monitored by SDS-PAGE followed by Coomassie Blue staining or immunoblot analysis. 1, Control; 2 and 3, two different preparations of IPTG-treated bacteria.
   B) Binding specificity of the IPTG-induced PBR in *Escherichia coli.* Specific binding of [³H]PK11195 (1.0 nM) was measured in the presence of the indicated concentrations of various ligands.
   C) Scatchard representation of the specific binding of ³H-PK11195 to IPTG-induced bacteria.

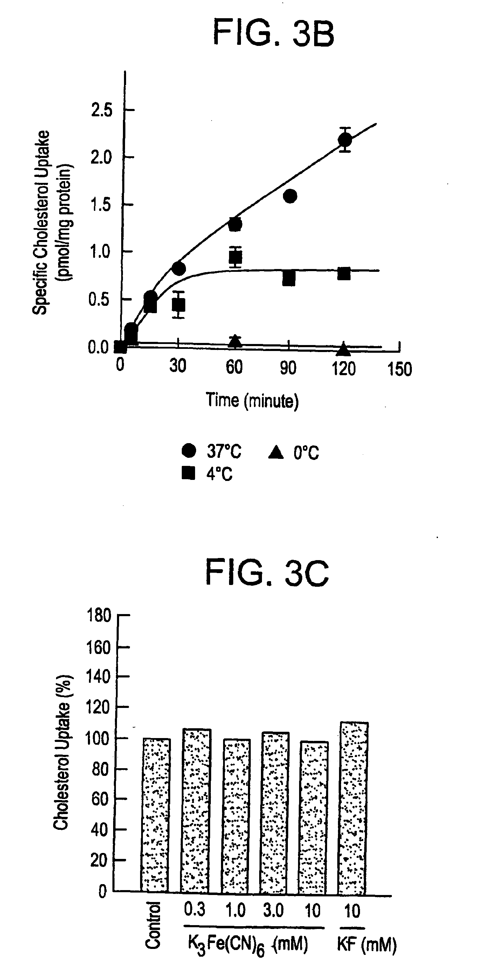

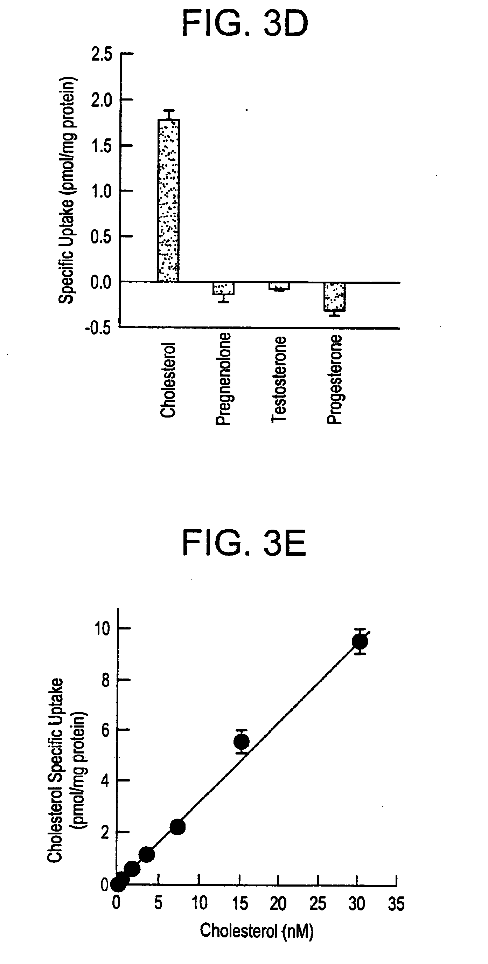

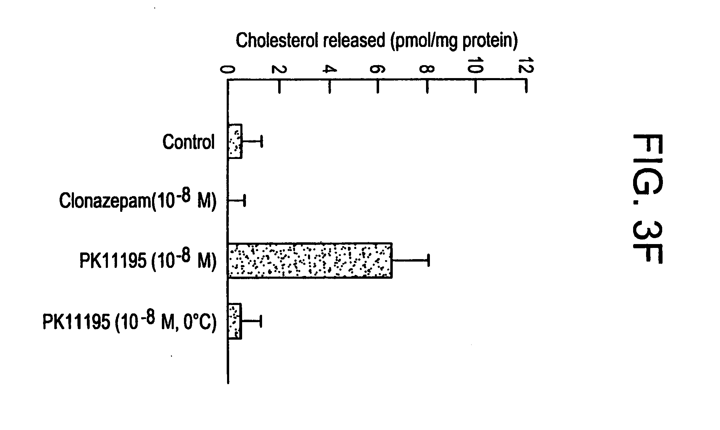

Figure 3. Characteristics of ³H-cholesterol uptake by *E. coli* cells.
   A) ³H-Cholesterol uptake by *E. coli* cells was examined using increasing concentrations of control or IPTG-treated transformed bacteria incubated in the presence of 6.7 nM ³H-cholesterol (50.0 Ci/mmol) for 60 min. at 37 C. Specific cholesterol uptake is defined as IPTG-induce minus basal values.
   B) ³H-Cholesterol specific uptake examined at the indicated temperatures using 100 ug bacterial protein.
   C) Effect of energy poisons on ³H-cholesterol uptake by IPTG-induced transformed bacteria.
   D) PBR expression induces uptake of cholesterol only. Bacteria were incubated under the same conditions as described above in the presence of the indicated radiolabeled steroid.
   E) ³H-cholesterol specific uptake determined in the presence of increasing concentrations of ³H-cholesterol.
   F) Ligand-induced release of cholesterol uptaken by the bacterial membranes. ³H-Cholesterol-labeled membrandes of IPTG-induced transformed bacteria were incubated with increased concentrations of PK11195 or clonazepam, and the ³H-cholesterol released was quantified. The results shown represent means ± SD from an experiment performed in triplicate. Similar results were obtained in three other independent experiments.

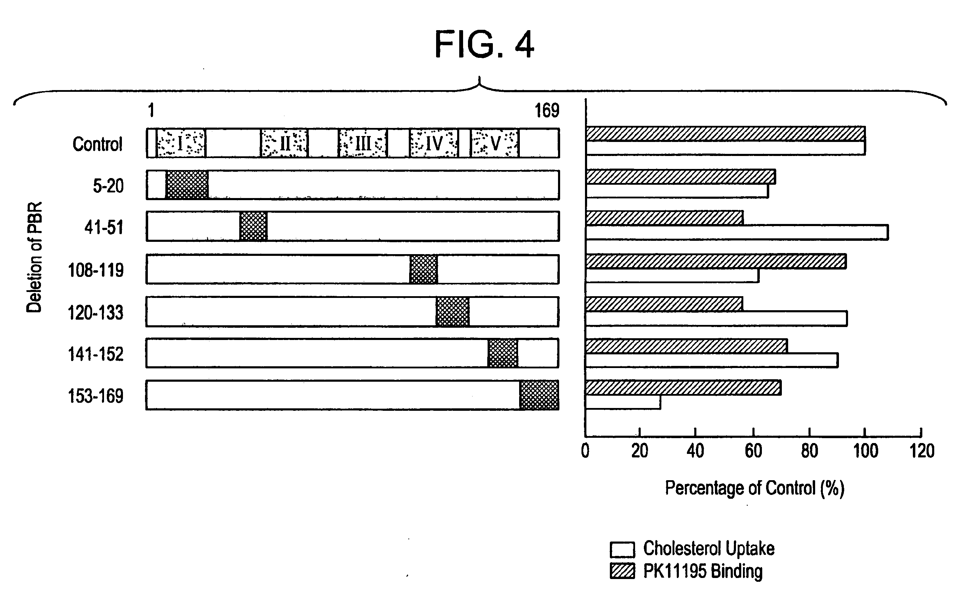

Figure 4. Deletion mutation analysis of PBR function-PK11195 ligand binding and ³H-cholesterol uptake by bacteria transfected with the wild-type and mutated PBRs. The expression of the proteins was induced by IPTG. Left, The five transmembrane regions of PBR are shown as well as the location of the deletions undertaken. Right, The effect of each deletion on PK11195 ligand binding and cholesterol uptake is shown. Results shown are the means of triplicates. 100% of PK 11195 ligand binding corresponds to 280 ± 22 fmol/mg protein. 100% of specific cholesterol uptake corresponds to 1.35 ± 0.15 pmol/mg of protein.

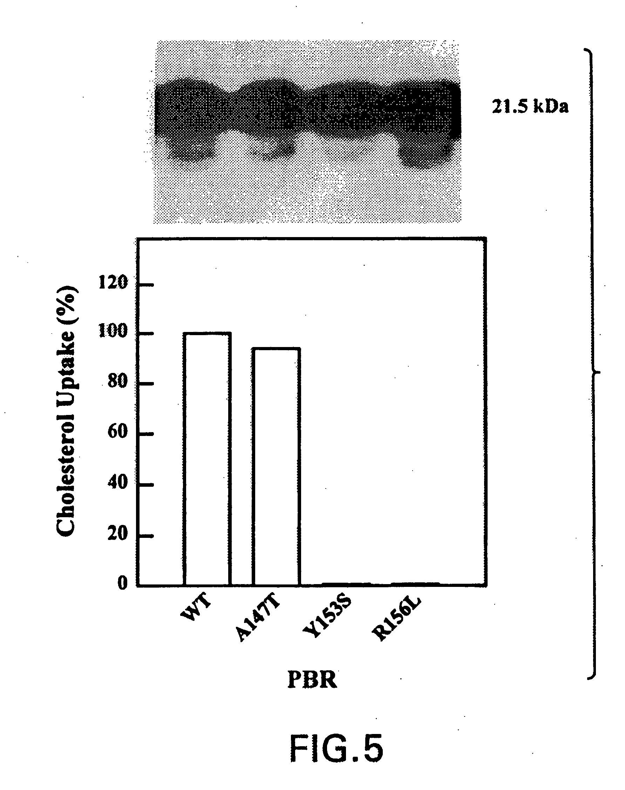

Figure 5. Identification of specific amino acids responsible for the uptake of cholesterol. ³H-Cholesterol uptake by bacteria transfected with the wild-type and point-mutated PBRs (bottom). The expression of the proteins was induced by IPTG. 100% of specific cholesterol uptake corresponds to 1.2 ± 0.1 pmol of cholesterol per mg of protein. Immunoblot analysis of the mutated PBRs expressed by the bacteria examined for ³H-cholesterol uptake, (top). Results shown are the means of triplicates.

### DETAILED DESCRIPTION

In one embodiment, the present invention relates to an isolated peptide having a cholesterol recognition/interaction amino acid sequence selected from the following sequences:
**Leu** Asn Tyr **Tyr** Val Trp **Arg** (SEQ ID NO:1)
**Leu** Asn **Tyr** Cys Val Trp **Arg** (SEQ ID NO:2)
**Leu** Asn **Tyr Arg** (SEQ ID NO:3)
**Val** Ala Tyr His Gln Tyr **Tyr** Gln **Arg** (SEQ ID NO:4)
and SEQ ID NO: 5

All peptides herein are written NH₂...COOH and the amino acids are the naturally occuring L isomers.

Cholesterol recognition/interaction amino acid sequences have been found in molecules shown or suggested to interact with cholesterol, such, as apolipoprotein A-1 (Boyle, T. P.and Marotti, K.R., 1992, *Gene* 117, 243-247), caveolin (Murata, M. *et al.* 1995, *Proc. Natl. Aca. Aci. USA* 92, 10339-10343), DBI (Papadopoulos, V. 1993, *Endocr. Rev.* 14, 222-240; Papadopoulos, V. 1998, *Proc. Soc. Exp. Biol. Med.* 217, 130-142), steroidogenesis acute regulatory protein (StAR) (Stocco, D. M. and Clark, B. J. 1996, *Endocr. Rev.* 17, 221-244), hedgehog protein (Porter, J. A. *et al.* 1996, *Science* 274, 255-259), cytochrome P450 C26/25 (Su, P. *et al.* 1990, *DNA Cell Biol.* 9-657-667), annexin II (Harder, T. *et al.* 1997, *Mol. Biol. Cell* 8, 533-545), sterol carrier protein-2 (Colles, S*.* M. *et al.* 1995, *Lipids* 30, 795-803), cholesterol 7α-monooxygenase (Kai, M. *et al.* 1995, *Lipid Res.* 36, 367-374), cholesterol oxidase (Ishizaki, T. *et al.* 1991, *J. Bacteriol.* 171, 596-601), cholesterol dehydrogenase (Horinouchi, S*. et al.* 1991, *Appl. Environ. Microbiol.* 57, 1386-1393), bile-salt-activated lipase precursor (cholesterol esterase) (Nilsson, *J. et al.* 1990, *Eur. J. Biochem.* 192, 543-550), and acyl-CoA cholesterol acyltransferase (Pape, M.E. *et al*. 1995, *J. Lipid Res.* 36, 823-838) as shown in Table 1.

**Table 1: Identification of cholesterol recognition/interaction amino acid sequences**

| | |
|---|---|
| Mouse PBR | *149-* **V**LNY**Y**VW**R** (SEQ ID NO:5) |
| Rat PBR | *150-* **L**NY**Y**VW**R** (SEQ ID NO:6) |
| Human PBR | *150-* **L**N**Y**CVW**R** (SEQ ID NO:7) |
| Bovine PBR | *149-* **L**N**YR** (SEQ ID NO:8) |
| Rat P450scc | *88*-**V**AYHQY**Y**Q**R** (SEQ ID NO:9) |
| Human P450scc | *88*-**V**AYHQY**Y**Q**R** (SEQ ID NO:10) |
| Pig P450scc | *88*-**V**AYHZH**Y**Q**K** (SEQ ID NO:11) |
| | |
| Mouse apolipoprotein A-I | *210-* **L**NE**Y**HT**R** (SEQ ID NO:12) |
| Mouse caveolin | *94-* **V**TK**Y**WFY**R** (SEQ ID NO:13) |
| Human hedgehog | *251-* **V**F**Y**VIET**R** (SEQ ID NO:14) |
| Mouse DBI | *25-* **L**FI**Y**SHF**K** (SEQ ID NO:15) |
| Mouse StAR | *6-* **L**CAGSS**Y**RHM**R** (SEQ ID NO:16) |
| Rat Annexin II | *145*-**V**YKEM**Y**KTDLE**K** (SEQ ID NO:17). |
| Rat P450c26/25 | *424-* **V**LCT**Y**VVS**R** (SEQ ID NO:18) |
| Strept. cholesterol oxidase | *420-* **V**SL**Y**LAIT**K** (SEQ ID NO:19) |
| Mouse cholesterol 7"-monooxygenase | *167*-**V**TEGM**Y**AFCY**R** (SEQ ID NO:20) |
| Nocardia cholesterol dehydrogenase | *91-* **V**TEA**Y**RQ**R** (SEQ ID NO:21) |
| Human Bile-Salt-Activated-Lipase | *226-* **L**SP**Y**NKGLI**R** (SEQ ID NO:22). |
| Rabbit acyl-CoA cholesterol acyltransferase | *96-* **V**VD**Y**IDEG**R** (SEQ ID NO:23) |

The presence of a cholesterol interaction/recognition amino acid sequence in the proteins listed in Table 1 signifies the likelihood that the proteins interact with cholesterol and provides insight into how these protein accomplish their functions in concert with cholesterol and how to alter these functions. For example, in the case of annexin which is a calcium-phospholipid/cholesterol actin binding protein important for various cell functions, i.e. from maintaining cell membrane shape and stability to facilitating the internalization of plasma membrane components, altering the cholesterol recognition/interaction site of annexin may result in destabilization and collapse of the cytoskeleton. This could be a useful method for targetting and destroying tumor cell growth.

After reviewing the description of the present invention, it would be within the skill of a person with ordinary skill in the art to design mutant proteins in which the cholesterol recognition/interaction amino acid sequence has been altered such that the protein's ability to interact/recognize cholesterol is reduced, increased, or abolished. Experiments on these mutants will further provide treatments for diseases associated with an altered, reduced, or increased function of these proteins, or even perhaps a method for targeting specific proteins through their ability to interact with cholesterol for altering the cholesterol recognition/interaction function for a desired purpose.

After reviewing the present disclosure, it will be evident to a person with ordinary skill in the art that it is possible to determine whether or not a protein interacts with cholesterol or recognizes cholesterol. This knowledge will provide insight into how a specific protein, whether known or yet to be discovered, functions or is regulated.

In another embodiment, the present invention provides a nucleotide sequence which encodes the peptides described above. The nucleotides corresponding to codons specifying the amino acids of the cholesterol recognition/interaction amino acid sequences described above are known to people in the art.

The generation of nucleic acid molecules encoding the cholesterol interaction/recognition amino acid consensus sequence(s) described above is routine for a person with ordinary skill in the art. Nucleic acid molecules of the present invention may be in the form of RNA, such as mRNA, or in the form of DNA, including, for instance cDNA and genomic DNA obtained by cloning or produced synthetically. The DNA may be double-stranded or single-stranded. Single-stranded DNA or RNA may be the coding strand, also known as the sense strand, or it may be the non-coding strand, also referred to as the anti-sense strand. Also included are chemically modified and substituted nucleic acids, e.g., those which incorporate modified nucleotide bases or which incorporate a labelling group.

The nucleic acid may be isolated. By "isolated" is meant a nucleic acid molecule, DNA or RNA, which has been removed from its native environment. For example, recombinant DNA molecules contained in a vector are considered isolated for the purposes of the present invention. Isolated RNA molecules include *in vivo* or *in vitro* RNA transcripts of the DNA molecules of the present invention. Isolated nucleic acid molecules according to the present invention further include such molecules produced synthetically.

Such isolated nucleic acid molecules are useful for probes for detecting a gene which contains this consensus sequence in a biological sample, for instance, by PCR, Southern blot, Northern blot, or other form of hybridization analysis as described in Molecular Cloning: A Laboratory Manual, 2nd edition, Sambrook, J. Fritsch, E.F. and Maniatis T., eds., Cold Spring Harbor Laboratory Press, Cold Spring Harbor, N.Y. (1989) or DNA Cloning, Volumes I and II (D. N. Glover ed., 1985) or Current Protocols in Molecular Biology Ausubel, F. M. *et al.* (Eds.) John Wiley & Sons, Inc. for general cloning methods). The entire disclosures of documents cited in this application are incorporated in their entirety by reference thereto. Typical nucleic acid probes may be prepared from the consensus amino acid sequences. In particular, probes may be prepared based upon segments of the amino acid sequence which possess relatively low levels of degeneracy, i.e., few or one possible nucleic acid sequences which encode therefor. These probes may further comprise a detectable group for easy identification and location of complementary sequences.

cDNA or genomic libraries of various types may be screened for new alleles or related sequences using the above probes. Phage or plasmid libraries may be used.

In addition to comprising a segment which encodes the peptides having the consensus sequence of the present invention, the nucleic acid of the present invention may also comprise a segment encoding a heterologous protein, such that the gene is expressed to produce the two proteins as a fusion protein.

Nucleic acid molecules of the present invention which encode the cholesterol recognition/interaction amino acid sequence of the present invention may include, but are not limited to those encoding the amino acid sequence of the consensus by itself; and additional coding sequences which code for additional amino acids, such as those which provide additional functionalities. Thus, the sequences encoding the consensus sequence may be fused to a marker sequence, such as a sequence encoding a peptide which facilitates purification of the fused polypeptide.

In addition to their use as probes, the nucleic acids of the present invention may also be used in the preparation of the peptides having the cholesterol recognition/interaction amino acid sequence of the present invention.

DNA encoding the peptides with the cholesterol recognition/interaction amino acid sequence of the present invention will typically be incorporated into DNA constructs capable of introduction to and expression in an *in vitro* cell culture. Often, the nucleic acids of the present invention may be used to produce a suitable recombinant host cell. Specifically, DNA constructs will be suitable for replication in a unicellular host, such as bacteria, e.g., *E. coli,* but may also be intended for introduction into a cultured mammalian, plant, insect or other eukaryotic cell lines. DNA constructs prepared for introduction into bacteria or yeast will typically include a replication system recognized by the host, the intended DNA segment encoding the desired polypeptide, transcriptional and translational initiation and termination regulatory sequences operably linked to the polypeptide encoding segment. A DNA sgement is operably linked when it is placed into a functional relationship with another DNA segment. For example, a promoter or enhancer is operably linked to a coding sequence if it stimulates the transcription of the sequence; DNA for a signal sequence is operably linked to DNA encoding a polypeptide if it is expressed as a preprotein that participates in the secretion of the polypeptide. Generally, DNA sequences that are operably linked are contiguous, and in the case of a signal sequence both contiguous and in reading phase. However, enhancers need not be contiguous with the coding sequences whose trancription they control. Linking is accomplished by ligation at convenient restriction sites or adapters or linkers inserted in lieu thereof. The selection of an appropriate promoter sequence will generally depend upon the host cell selected for the expression of the DNA segment. Examples of suitable promoter sequences include prokaryotic, and eukaryotic promoters well known in the art and include promoters such as the *trp, lac* and phage promoters, tRNA promoters and glycolytic enzyme promoters, T7 promoter, T3 promoter, SP6 promoter, SV40 promoter, CMV promoter and MoMLV LTR are known and available. See Sambrook *et al.,* 1989, *supra.* The transcriptional regulatory sequences will typically include a heterologous enhancer or promoter which is recognized by the host.

Expression vectors useful in the present invention include chromosomal-, episomal- and virus-derived vectors, e.g., vectors derived from bacterial plasmids, bacteriophage, yeast episomes, yeast chromosomal elements, viruses such as baculoviruses, papova viruses, vaccinia viruses, adenoviruses, fowl pox viruses, pseudorabies viruses and retroviruses, and vectors derived from combinations thereof such as cosmids and phagemids.

Conveniently available expression vectors which include the replication system and transcriptional and translational regulatory sequences together with the insertion site for the consensus sequence peptide encoding segment may be employed. Among vectors preferred for use in bacteria include pBS vectors, Phagescript vectors, Bluescript vectors commercially available. Among preferred eukaryotic vectors are pSV2CAT, pWLNEO, available from Stratagene; and pSVK2, pMSG available from Pharmacia. Other suitable vectors will be readily apparent to the skilled artisan such as pET vectors, such as pET15b, pZeoSV2, pCMV and the like. Examples of workable combinations of cell lines and expression vectors are described in Sambrook *et al.* and in Metzger *et al., Nature* 334, 31-36, 1988. For example, where an insect host cell is selected as the host cell of choice to express the polypeptide, the cDNA encoding the polypeptides of the invention may be cloned into a baculovirus expression vector (pV-IKS). The recombinant baculovirus may then be used to transfect a suitable insect host cell, e.g. SF9 cells, which may then express the polypeptide. See, e.g. D.D. Morrison *et al.,* Cell 58, 649-657, 1989, M.D. Summers and G.E. Smith, A Manual of Methods for Baculovirus Vectors and Insect Cell Culture Procedures, Texas Agricultural Station, College Station, Tex., 1987.

The vectors containing the DNA segments of interest, e.g. those encoding the peptides comprising the cholesterol recognition/interaction amino acid sequence of the present invention, can be transferred into the host cell by well known methods, which may vary depending upon the type of host used. For example, calcium chloride transfection is commonly used for prokaryotic cells, whereas calcium phosphate treatment may be used for other hosts. Introduction of the construct into the host cell can be effected by DEAE-dextran mediated transfection, cationic lipid-mediated transfection, electroporation, transduction, infection, or other methods. See Davis et al. Basic Methods in Molecular Biology, 1986. The term "transformed cell" as used herein, includes the progeny of originally transformed cells.

In another embodiment, the present invention provides a cell stably transfected with a vector comprising the cholesterol interaction/recognition amino acid sequence of the present invention. The term "transfected" as used herein refers to cell having undergone the process of introduction of nucleic acid or a nucleic acid vector into a cell. Various methods of transfecting a cell are possible including microinjection, CaPO₄ precipitation, lipofection (liposome fusion), electroporation and use of a gene gun. The term "stable" as used herein refers to the introduction of a gene into the chromosome of the targeted cell where it integrates and becomes a permanent component of the genetic material in that cell. A transfected cell containing a vector having a peptide comprising the consensus sequence may only be transiently transfected, resulting in transient expression of the peptide. The term "transient" as used herein relates to the introduction of a gene into a cell to express a cholesterol consensus containing peptide, where the introduced DNA is not integrated into the host cell genome and is accordingly eliminated from the host cell over a period of time. Transient expression relates to the expression of a product during a period of transient transfection. An episomal transfection is a variant of stable transfection in which the introduced gene is not incorporated in the host cell chromosomes but rather is replicated as an extrachromosomal element. This can lead to apparently stable transfection of the characteristics of a cell.

A cell may be transfected with a vector containing a selectable marker or cotransfected with a second vector containing the desired selectable marker. This selectable marker is used to select those cells which have become transfected. Types of selectable markers which may be used are well known to those of ordinary skill in the art.

In another embodiment, there is provided a transfected cell wherein a peptide comprising the cholesterol recognition/interaction amino acid sequence of the present invention is expressed as a cell surface peptide. By "cell surface" peptide is meant a peptide which wholly or partially spans the cell membrane, and which is exposed on the surface of the cell. The peptide comprising the cholesterol recognition/interaction amino acid sequence of the present invention can be expressed as a secreted peptide. By "secreted peptide" is meant a peptide which is not associated with the cell membrane, but rather is intracellularly processed for secretion into the extracellular environment or other cellular compartment.

The peptide comprising the cholesterol recognition/interaction amino acid sequence of the present invention can be recovered and purified from recombinant cell cultures by well-known methods including ammonium sulfate or ethanol precipitation, acid extraction, anion or cation exchange chromatography, phophocellulose chromatography, hydrophobic interaction chromatography, affinity chromatography, hydroxylapatite chromatography, lectin chromatography, and high performance liquid chromatography ("HPLC") is employed for purification. Polypeptides of the present invention include naturally purified products, products of chemical synthetic procedures, and products produced by recombinant techniques from a prokaryotic or eukaryotic host, including, for example, bacterial, yeast, higher plant, insect and mammalian cells.

It is another aspect of the invention to provide a transgenic plant comprising in its genome a genetic construct comprising a nucleic acid encoding PBR with an altered cholesterol recognition/interaction sequence wherein the ability of PBR to interact with cholesterol and channel it into the cell is reduced or abolished, thereby resulting in a reduced or abolished uptake of cholesterol in the cell, and/or reduced or abolished production of steroids in the transgenic plant. This method may offer an opportunity to regulate cholesterol levels in plants, an well as specific plant steroids such as cardenolides, etc. required for nutrition or any other purpose. Research has demonstrated that transgenic plants are capable of passing on the inserted genes to their progeny by normal Mendelian inheritance (Christou et al., 1990, *Trends in Biotechnol.* 8, 145-151). All such progeny plants which inherit the inserted genetic construct are also transgenic plants as the term is used here.

The production of transgenic plants is known in the art. Please see for example, U.S. Patent No. 5,869,720 to John, M.E. issued on February 9, 1999, and U.S. Patent No. 5,859,347 to Brown et al. issued on January 12, 1999. The disclosures of both patents are hereby incorporated in their entirety by referene thereto.

Briefly, the nucleic acid desired can be inserted into a suitable plant transformation vector for transformation into the desired plant species. Suitable plant transformation vectors include those derived from a *Ti* plasmid of *Agrobacterium tumefaciens.* A plant transformation vector preferably includes all of the necessary elements needed for transformation of plants, or plant cells. Specific plant tissues can be targeted by using promoters specific for expression in fruit, fiber, root, etc... Promoters used can be inducible by plant hormone such as ecdysone, by antibiotic, such as tetracycline, by changes in temperature, such as heat shock elements. Typical plant transformation vectors comprise selectable marker genes, one or both of the T-DNA borders, cloning sites, appropriate bacterial genes to facilitate identification of transconjugates, broad host-range replication and mobilization functions and other elements as desired.

Transformation of plant cells may be effected by delivery of a transformation vector or of free DNA by use of a particle gun which comprises high velocity microprojectiles coated with the vector into plant tissue. Selection of transformed plant cells and regeneration into whole plants may be carried out using conventional procedures. Other transformation techniques capable of inserting the desired gene or nucleic acid into plant cells may be used, such as eletroporation or chemicals that increase free nucleic acid uptake. Illustrative examples of methods suitable for regenerating transgenic plants are : corn (Fromm et al., 1990, *Bio*/*Technology* 8:833-839; and Gordon-Kamm et al., 1990 *The Plant Cell* 2:603-618); rice (Wang et al., 1988, *Plant Mol. Biol.* 11:433-439) and wheat (Vasil et al., *Bio*/*Technology* 8:743-747).

In one aspect, the transformed or transfected cells are useful for screening agents or drugs which alter the ability of polypeptides comprising the cholesterol recognition/interaction amino acid sequence and mutants of said sequence to interact, recognize or bind to cholesterol or its derivatives. An *in vitro* system for determining whether a compound, drug, or agent is an agonist or antagonist of the ability of the polypeptide comprising the cholesterol recognition/interaction amino acid sequence of the present invention to interact with cholesterol can be designed. A polypeptide comprising the cholesterol recognition/interaction amino acid sequence of the present invention can be incubated along with cholesterol under conditions, i.e. salts, pH, lipid, ions, where interaction between cholesterol and the peptide comprising the cholesterol recognition/interaction amino acid sequence occurs, The complex may then be incubated with a test compound. Interaction between the polypeptide and cholesterol may then be measured. An increase or decrease in the level of interaction between the polypeptide and cholesterol in response to a particular compound would indicate that the compound is an agonist or antagonist of that binding, respectively.

The same test can be administered on cells which express a polypeptide comprising the cholesterol interaction/recognition amino acid sequence. The pH and temperature which are most effective for cholesterol interaction are preferably used but it is possible to replicate the conditions found in a diseased cell for testing the effect of a particular drug with respect to a proposed therapy for the disease.

A test compound may be a chemical compound, a mixture of chemical compounds, a biological macromolecule, or an extract made from biological materials such as bacteria, plants, fungi, or animal cells or tissues.

For many of the methods of the present invention, the polypeptides and nucleic acids of the invention may be covalently attached or linked to a detectable group to facilitate screening and detection. Useful detectable groups or labels, are generally well known in the art. For example, a detectable group may be a radiolabel, such as, ¹²⁵I, ³²P, or ³⁵S, or a fluorescent or chemiluminescent group. Alternatively, the detectable group, may be a substrate, cofactor, inhibitor, affinity ligand, antibody binding epitope tag, or an enzyme which is capable of being assayed. Suitable enzymes include, e.g., horseradish peroxidase, luciferase, or another readily assayable enzymes. These enzyme groups may be attached to the polypeptide comprising the consensus sequence by chemical means or expressed as a fusion protein as already described.

In another embodiment, the present invention provides a method for detecting presence, absence, increase or decrease of cholesterol in a biological sample. This can be done by immobilizing the polypeptide comprising the cholesterol recognition/interaction amino acid sequence upon a solid support, e.g., a microtiter well, or nitrocellulose membrane. The sample to be assayed is exposed to the immobilized polypeptide, and the amount of cholesterol-bound polypeptide is measured.

The solid support can include agarose, cellulose, dextran, Sephadex, Sepharose, carboxymethyl cellulose, polystyrene, filter paper, nitrocellulose, ion exchange resins, plastic films, glass beads, polyaminemethylvinylether maleic acid copolymer, amino acid copolymer, ethylene-maleic acid copolymer, nylon, silk, etc. The support may be in the form of, e.g., a test tube, microtiter plate, beads, test strips, or the like. The reaction of the solid support with the polypeptide may be carried out by methods well known in the art.

By "biological sample" is intended any biological sample obtained from an animal, cell line, tissue culture, plant, insect, or other source which may contain cholesterol or its derivatives, or cholesterol interacting/recognizing polypeptides mRNA or DNA. Biological samples include body fluids (such as saliva, blood, plasma, urine, mucus, synovial fluid, etc.) tissues (such as muscle, skin, and cartilage) and any other biological source suspected of containing cholesterol or derivatives of cholesterol, or cholesterol binding polypeptides or nucleic acids. Methods for obtaining biological samples such as tissue are well known in the art.

In another embodiment, the present invention provides a method for identifying whether or not a protein recognizes or interacts with cholesterol said method comprising identifying the presence or absence of the above-mentioned cholesterol recognition/interaction amino acid sequence by analyzing the cholesterol interaction/recognition amino acid sequence of the unknown protein as discussed in the examples below. The presence of the amino acid sequence of the present invention is an indication of the likelihood that the protein recognizes/interacts with cholesterol.

In another embodiment, the present invention provides a method for conferring to a molecule the ability to interact with cholesterol which is recognized by the cholesterol recognition/interaction amino acid sequence. Since the consensus sequence for interaction/recognition of cholesterol has been elucidated, it is now possible to introduce into a molecule or polypeptide, natural or synthetic, a cholesterol recognition/interaction sequence such that the protein or polypeptide, natural or synthetic, is now able to recognize/interact with cholesterol or a cholesterol derivative. The consensus sequence can be introduced at the DNA or RNA level, by inserting the nucleotide sequence encoding the consensus - sequence into a part of the gene for said molecule, such that the consensus sequence is translated along with the molecule and together they form a fusion protein. Preferably, the consensus sequence is inserted at the amino or carboxy terminal of the gene such that secondary and tertiary folding of the gene product does not inhibit the interaction of the consensus sequence with cholesterol. The fusion protein can be tested for its ability to interact/recognize or bind cholesterol or a cholesterol derivative.. These fusion proteins can be used for therapy or diagnostics. The molecule into which the cholesterol interaction/recognition sequence is desired can be natural or synthetic.

In yet another embodiment, the present invention provides a molecule which blocks the cholesterol interaction/recognition ability of a peptide comprising the cholesterol recognition/interaction amino acid sequence described above to recognize/interact with cholesterol. The molecule can be an antibody, a peptide, or drug. Antibody production using peptides is well known in the art, see, *e.g.,* Sutcliffe, *et al. Science* 219, 660-666, 1983; Wilson *et al., Cell* 37, 767-778, 1984, and Bittle *et al., J. Gen. Virol.* 66, 2357-2354, 1985. As used herein, the term "antibody" (AB) or "monoclonal antibody (Mab) is meant to include intact molecules, single chain whole antibodies, and antibody fragments. Antibody fragments of the present invention include Fab and F(ab')2 and other fragments including single chain Fvs (scFv) and disulfide-linked Fvs (sdFv). Also included in the present invention are chimeric and humanized monoclonal antibodies and polyclonal antibodies specific for the polypeptides of the present invention. The antibodies of the present invention may be prepared by any of a variety of methods. For example, cells expressing a polypeptide of the present invention or an antigenic fragment thereof can be administered to an animal in order to induce the production of sera containing polyclonal antibodies. Monoclonal antibodies can be prepared using hybridoma technology known in the art see, e.g., Harlow *et al.,* Antibodies: A Laboratory Manual (Cold Spring Harbor Laboratory Press 2nd ed., 1988). Additionally, antibodies capable of binding to a polypeptide antigen of the present invention may be produced through the use of ariti-idiotypic antibodies. Such a method makes use of the fact that antibodies are themselves antigens, and that, therefore, it is possible to obtain an antibody which binds to a second antibody.

Additionally, the cholesterol interaction/recognition peptide may be useful in modeling small molecules which interfere with cholesterol binding *in vivo.* In particular, the structure of the cholesterol interaction/recognition domain from the known amino acid sequence and the 3-dimensional structure, which may be determined by x-ray crystallographic methods known in the art, may be applied in generating synthetic analogs, cholesterol derivatives, and mimics of the particular cholesterol interaction/recognition domain. Synthetic elements may be pieced together based upon their analogy to the structural and chemical aspects of the cholesterol interaction/recognition domain. Such mimics, analogs, and derivatives may be used in blocking or inhibiting specific functions resulting from cholesterol binding to a peptide or gene product. These functions can vary since a role for cholesterol has been implicated in cell signaling, cell proliferation, gene regulation, cytoskeletal anchoring and stability, neural transmission, fertility, stress, diabetes, stroke, to name a few, and may thus be useful as therapeutic treatments according to the methods described herein.

In another embodiment; the present invention relates to a method for altering the cholesterol binding ability of molecules which contain the cholesterol recognition/interaction amino acid sequence described above, comprising altering said site such that cholesterol recognition is reduced or eliminated.

We have been able to produce PBR with reduced or no ability to recognize and interact with cholesterol by modifying the cholesterol recognition/interaction site of PBR, for example by changing amino acid 153 from tyrosine to serine; or by changing amino acid 155 from arginine to leucine. These modifications are expected to produce similar results in species which express PBR, and on proteins other than PBR which contain the consensus sequence. Therefore, it would be possible to alter the cholesterol recognition/interaction ability of a polypeptide comprising the peptide consensus sequence of the present invention by changing the tyrosine in the consensus sequence to serine or other amino acid with no charge, for example, or changing the consensus sequence arginine to leucine or any other amino acid without a charge.

In this application is described the cholesterol interaction/recognition amino acid sequence first discovered on PBR. In addition to the presence of PBR in the mitochondrial membrane, PBR is present in the plasma membrane (Oke, B. O. *et al.* 1992, *Mol. Cell. Endocr.* 87:R1-R6; Garnier, M. *et al.* 1993, Endocrinology 132:444) and in the nuclear membrane (Hardwick, M. *et al.* 1999, *Cancer Research* 59:831-842). Results reported in this application indicate that the carboxy terminal of the receptor is responsible for the interaction and subsequent uptake of cholesterol. Cholesterol is released from the receptor once a ligand binds to the amino terminal portion of the receptor. In the case of the mitochondria, the cholesterol is released into the inner mitochondrial membrane where it is available for interaction with P450scc and is cleaved to produce pregnenolone in steroidogenic cells. Pregenenblone is the precursor of steroids.

In addition to the above described uses, the polypeptides and nucleic acids of the present invention may also be used in therapeutic applications for the treatment of human or non-human mammalian patients.

The nucleic acid encoding the polypeptide comprising the cholesterol interaction sequence of the present invention can be used as a cholesterol acceptor in order to regulate the amount of cholesterol in a tissue or blood. The nucleic acid is administered alone or as part of a vector such that it is translated, and the resulting polypeptide is capable of interacting with/binding to cholesterol. The nucleic acid sequence encoding a peptide comprising the cholesterol interaction/recognition consensus sequence can be administered prophylactically, or to patients having a disease or condition characterized by an elevated plasma cholesterol level. By "elevated level" is meant a higher level relative to what is normally found in the plasma. Administration can be by exogenous delivery of the nucleic acid as naked DNA, DNA associated with specific carriers, or in a nucleic acid expression vector to a desired tissue by means of an appropriate delivery vehicle, e.g. a liposome, by use of iontophoresis, eletroporation and other pharmacologically approved methods of delivery. Some methods of delivery may include: encapsulation in liposomes, transduction by retroviral vectors, localization to nuclear compartment utilizing nuclear targeting site found on most nuclear proteins, transfection of cells *ex vivo* with subsequent reimplantation or administration of the transfected cells, a DNA transporter system. Intravenous administration with a drug carrier designed to incrase the circulation half-life of the nucleic acid encoding the cholesterol interaction/recognition sequence can be used. Additionally, the treatment of any disorder may comprise gene therapy techniques involving the cholesterol interaction/recognition domain sequence or a mutation or alteration of the cholesterol interaction/recognition domain sequence. Strategies for gene therapy are reviewed in Friedman, *Science* 244, 1275, 1989.

Drug delivery vehicles are effective for both systemic and topical administration. They can be designed to serve as a slow release reservoir, or to deliver their contents directly to the target cell. An advantage of using direct delivery drug vehicles is that multiple molecules are delivered per uptake. Such vehicles have been shown to increase the circulation half-life of drugs which would otherwise be rapidly cleared from the blood stream. Some examples of such specialized drug delivery vehicles are liposomes, hydrogels, cyclodextrins, biodegradable nanocapsules, and bioadhesive microspheres.

The nucleic acid sequence encoding a cholesterol recognition/interaction sequence may also be systemically administered. Systemic absorption refers to the accumulation of drugs in the blood stream followed by distribution throughout the entire body. Administration routes which lead to systemic absorption include: intravenous, intrmuscular, subcutaneous, intraperitoneal, intranasal, intrathecal and ophthalmic. A gene gun may also be utilized. The dosage will depend upon the disease indication and the route of administration but should be between 1-1000 ug/kg of body weight/day. The duration of treatment will extend through the course of the disease symptoms, possibly continuously. The number of doses will depend upon disease delivery vehicle and efficacy data from clinical trials.

The nucleic acid comprising the cholesterol interaction/recognition consensus sequence may be administered utilizing an *in vivo* approach whereby the nucleic acid will be administered directly to an animal by intravenous injection, intramuscular injection, or by catheterization and direct delivery of the nucleic acid via the blood vessels and directed to a target organ by using tissue-specific promoters.

The polypeptides of the present invention may be used to inhibit or block uptake of cholesterol into cells bv competing for cholesterol. These methods may generally be used in the treatment of a variety of diseases resulting from an increase in cholesterol in the cell or in the plasma, or in screening compounds effective for such treatment. Cholesterol is required for normal cell growth and proper membrane structure and function. Unregulated accumulation of cholesterol is cytotoxic and a failure to maintain cholesterol homeostasis results in a number of pathological states (See: Subcellular Biochemistry vol. 28, *Cholesterol: its Functions and Metabolism in Biology and Medicine.* Bittman, Robert (Ed), Plenum Press, N.Y.). Specific disorders include gallstones, atherosclerosis, Niemann-Pick C, Sitosterolemia, Dystrophy, Tumor proliferation (tumorigenesis), Schnyder's corneal crystalline dystrophy. Brain disorders include cholesterol metabolism and Alzheimer's disease, Tellurium toxicity, Smith-Lemli-Opitz syndrome, myelinization, developmental abnormalities and demyelization : Charcot-Marie-Tooth disease; Pelizaeus-Merzbacher disease, Multiple sclerosis, SLA, to name a few. Alternatively, the methods and compositions may be useful as prophylactic treatment, or in screening for compounds effective in prophylactic treatments.

In another aspect of the invention, the peptide comprising the cholesterol recognition/interaction amino acid sequence of the present invention can be used to deliver, when needed, cholesterol, or a cholesterol derivative which binds the cholesterol recognition/interaction sequence of the present invention. The peptide can be complexed to cholesterol prior to administration, thereby avoiding the nonspecific attachment of cholesterol to other factors such as albumin for example.

The quantities of reagents necessary for effective therapy, also referred to herein as an "effective amount", or "therapeutically effective amount", will depend upon many different factors, including means of administration, target site, Physiological state of the patient and other mdicants administered. Thus, treatment doses will need to be titrated to optimize safety and efficacy. Typically, dosages used *in vitro* may provide useful guidance in the amounts useful for in situ administration of these reagents. Animal testing of effective doses for treatment of particular disorders will provide further predictive indication of human dosage. Generally, therapeutically effective amounts of the polypeptide comprising the cholesterol interaction/recognition domain of the present invention will be from about 0.0001 to about 100 mg/kg, and more usually, from about 0.001 to about 0.1 mg/kg of the subject's body weight. Various considerations are described, e.g, in Gilman *et al.,* (Eds.), Goodman and Gilman's: The Pharmacological Basis of Therapeutics, (8th ed., 1990), Pergamon Press, and Remington's Pharmaceutical Sciences (7th ed., 1985) Mack Publishing Co., Easton, Pa. Methods of administration, also discussed in the above references, include, e.g., oral, intravenous, intrperitoneal or intramuscular administration, and local administration, including topical, transdermal diffusion and aerosol administration, for therapeutic, and/or prophylactic treatment. The active agent will generally be administered in a composition additionally comprising a pharmaceutically acceptable carrier. Pharmaceutically acceptable carriers will include water, saline, buffers, and other compounds described in, e.g., the Merck Index, Merck and Co., Rahway, N.J.

By "subject" is meant fish, animals, including plants, insects, monkeys, apes, cats, dogs, birds, cows, pigs, mice, horses, rabbits and humans.

Constituents of pharmaceutical compositions, in addition to the active agents, include those generally known in the art for the various administration methods used. For example, oral forms generally include powders, tablets, pills, capsules, lozenges and liquids. Similarly, intravenous, intraperitoneal or intrmuscular formulations will generally be dissolved or suspended in a pharmaceuticlly acceptable carrier, e.g., water, buffered water, saline, and the like. Additionally, these compositions may include additional constituents which may be required to approximate physiological conditions, such as pH adjusting and buffering agents, toxicity adjusting agents, wetting agents and the like. For solid compositions, conventional nontoxic solid carriers may be used which include, e.g., pharmaceutical grades of mannitol, lactose, starch, magnesium stearate, sodium saccharin, talcum; cellulose, glucose, sucrose, magnesium carbonate, and the like.

Administration may also be carried out by way of a controlled release composition or device, whereby a slow release of the active ingredient allows continuous administration over a longer period of time.

The following MATERIALS AND METHODS were used in the examples that follow.

*MA-10 Leydig cells-* MA-10 mouse Leydig tumor cells were maintained as we previously described (Papadopoulos, V. *et al.* 1990, *J. Biol. Chem.* 265:3772-3779). For the cholesterol uptake assays, mitochondria were isolated as we described (Papadopoulos *et al.* 1990, *supra;* Krueger and Papadopoulos, 1990, *supra*) and resuspended in buffer A (250mM Sucrose, 20mM KCl, 15mM trietholamine hydrochloride [pH7.0], 10mM K₃PO₄ and 10mM MgCl₂) at 1mg/ml total protein concentration (Leaver, H. A. and Boyd G. S. 1981, *J*. *Endocrinol.* 91:123-133). Mitochondria were then incubated with [1,2-³H]-cholesterol (0.127 uCi/100 nmol) in 0.3 ml buffer A at 37 °C (or the indicated temperature) for the indicated time period. At the end of the incubation, steroids were extracted and ³H-pregnenolone formed was isolated by thin layer chromatography and quantified (Amri, H. *et al.* 1996, *Endocrinology* 137:5707-5718).

*Construction of the mouse PBR expression* vector *(pET15bPBR) and Expression of Recombinant PBR in bacteria-* The pET. system (Novagen, Madison, WI) was used to express the MA-10 mouse PBR (mPBR) recombinant protein. The insert containing full length coding sequence as well as the NdeI and XhoI site extensions at the 5' and 3' ends were generated by PCR using the following primers: ATATATACATATGCCTGAATCCTGGGTG (SEQ ID NO:24) and ATACTCGAGTGGGTGCCTTCACTCTG (SEQ ID NO:25), respectively. The MA-10 full length PBR cDNA (Garnier, M. et *al.* 1994, *Mol. Pharm.* 45:201-211, was used as template and encodes the amino acid sequence ATVLNYYVWRDNS. This mPBR fragment was inserted into pET1 55 vector and linearized with NdeI and XhoI downstream of the T71ac promoter. Recombinant mPBR expression vector was used to transform the BL21(DE3) *Escherichia coli* strain (Novagen) where the expression of recombinant mPBR protein was' induced by 1mM. isopropyl-1-thiol-β-D-galactopyranoside (IPTG). PBR protein expression was monitored by SDS-PAGE followed by Coomassie Blue staining or immunoblot analysis using anti-PBR antiserum (Amri *et al.* 1996, *supra*). Binding specificity of the IPTG-induced PBR in *E. coli* was determined in binding studies where specific binding of ³H-PK 11195 (1.0 nM) was measured in the presence of the indicated concentrations of the indicated ligands.

³H-Cholesterol uptake by *E. coli* cells was examined using the indicated concentrations of control or IPTG-treated transformed bacteria incubated in the presence of 6.7 nM ³H-cholesterol (50.0 Ci/mmol) for 60 min at 37°C. Specific cholesterol uptake is defined as IPTG-induced minus basal values, *E. coli* protoplasts were prepared from cells grown in LB at 37°C to the logarithmic phase of growth and centrifuged at 10,000g for 5 min. The cells were washed twice in 10mM Tris-HCl buffer [pH8.0] and the pellet was resuspended in a solution containing 20%(w/w) sucrose and 0.1M Tris-HCl [pH8.0]. The cells were then suspended in 1ml of buffer per 10 OD₄₅₀ and mixed. Within 1 min lysozyme was added from a 2mg/ml stock solution in distilled water to a final concentration of 100 ug/ml at 37°C, and stirring was continued for the next 12 min. The cell suspension was diluted 1:10 with 0.1M Na₂EDTA prewarmed to 37°C. Continuous stirring and slow dilution over 2.5 min prevented cell lysis. More than 99% of the cells became spherical within 10 minutes (Weiss, R. L. 1978, *Meth. Cell Biol.* 20:141-147). Protoplasts (100 ug in 0.3 ml final volume) were then incubated in the presence of 6.7 nM ³H-cholesterol (50.0 Ci/mmol) for 60 min at 37°C for cholesterol uptake assays. ³H-Cholesterol-labeled membranes of IPTG-induced transformed bacteria, incubated with 30 nM ³H-cholesterol, were treated with PK 11195 or clonazepam and the ³H-cholesterol released was quantified by liquid scintillation spectrometry.

*Site-directed Mutagenesis-* Mutations were performed using the QuikChange Site-Directed Mutagenesis kit from Stratagene (La Jolla, CA). Briefly, miniprep pET-PBR plasmid dsDNA was used as template. Synthetic oligonucleotide primer pairs containing A147T, Y153S, R155L point mutations and PBR deletions, Δ5-20, Δ41-51, Δ108-119, Δ120-133, Δ141-152, Δ153-169, each complementary to the opposing strand of the vector, were extended during temperature cycling by pfu DNA polymerase. Upon incorporation of the oligonucleotide primers, mutated plasmids containing staggered nicks were generated. After temperature cycling, the products were treated with *Dpn* I is used to digest the parental DNA template and select for the generated mutation. The nicked vector DNAs containing the desired mutations were then transformed into *E. coli.* The mutated plasmids were prepared by ABI Prism Miniprep Kit. The generated mutations and deletions were confirmed by sequencing using the ABI Prism Dye Terminator Cycle Sequencing ready reaction kit (Perkin Elmer Applied Biosystems, Foster City, CA). DNA sequencing was performed at the Lombardi Cancer Center Sequencing Core Facility (Georgetown University).

*Radioligand binding assays-* ³H-1- (2-chlorophenyl)-N-methyl-N-(1-methyl-propyl)-3-isoquinolinecarboxamide (PK 11195) binding studies were performed as we previously described (Papadopoulos, V. *et al.* 1990, *J. Biol. Chem.* 265: 3772-3779; Garnier, M. *et al.* 1994, *Mol. Pharm.* 45:201-211). The dissociation constant (Kd) and the number of binding sites (Bmax) were determined by Scatchard plot analysis of the data using the LIGAND program (Munson, P. J. and Rodbard, D. 1980, Anal. *Biochem.* 107:220-239).

*Protein* measurement- Microgram amounts of protein were quantified using the dye-binding assay of Bradford (Bradford, M. M. 1976, *Anal. Biochem.* 72:248-254) using bovine serum albumin as the standard.

*Statistics-* The results shown represent the means ± S.D. or S.E.M. from 2 to 6 independent experiments. Statistical analysis was performed by ANOVA followed by the *Student-Newman-Keuls* test or the *Dunnett* multiple comparisons test using the Instat (v.2.04) package from GraphPad, Inc. (San Diego, CA).

### Example 1

A three-dimensional model of human PBR was built using molecular dynamics simulations and shown to accommodate a cholesterol molecule within its five helices (Bernassau, J. M. *et al.* 1993, *J. Mol. Graph.* 11:236-245). Despite differences in the primary amino acid sequence between the human and mouse PBR, similar data was obtained when the mouse 18 kDa PBR protein was submitted to the same analysis (Papadopoulos, V. 1996, *supra*), further suggesting that PBR may function as a channel for Cholesterol. In order to test this hypothetical model we used two cell model systems:
(i) the MA-10 Leydig cells, a steroidogenic cell model which expresses high levels of PBR (-40 pmol/mg protein; Papadopoulos, V. *et al.* 1990, *J. Biol. Chem.* 265:3772-3779), and as all eukaryotic cells contains endogenous cholesterol; and (ii) the E.coli DE3 cells which do not express PBR (this study), do not have endogenous cholesterol (Moat, A.G. and Foster, J.W. 1995, Microbial Physiology. Wiley-Liss, New York.) and do not form steroids. Thus, using these cell models we attempted to correlate PBR expression with the cholesterol transport function. In addition, we used a method with radiolabeled cholesterol (Leaver, H. A. and Boyd G. S., 1981, *J. Endocrinol.* 91:123-133) to quantify cholesterol movement. This method allows for the distinction between the exogenously supplied cholesterol and the endogenous cholesterol, permitting an easy quantification, and direct measurement of the pregnenolone formed, in the case of the steroidogenic cells where cholesterol transported to IMM is cleaved by the P450scc to generate pregnenolone. Data shown in Fig. 1 validate the use of this method. Fig.1 shows that in the steroidogenic Leydig cells, ³H-cholesterol uptake by the mitochondria and transport from OMM to IMM were stimulated by specific PBR ligands resulting in increased ³H-pregenenolone formation. These data are in agreement with previous findings in all steroid synthesizing cell types of the body (Papadopoulos, V. 1993, *Endocr. Rev.* 14:222-240;Papadopoulos, V. 1998, *Proc. Soc. Exp. Biol. Med.* 217:130-142). Moreover, a similar PBR-dependent cholesterol transport mechanism from OMM to IMM was recently identified in liver mitochondria (Tsankova, V. *et al.,* 1995, *Eur. J. Pharm.* 294:601-607). Cholesterol transport to liver IMM may be required for cholesterol detoxification from the periphery by the IMM sterol-27-hydroxylase (Tsankova *et al.,* 1995, *supra*). Interestingly, the rate of cholesterol uptake and intramitochondrial transport to IMM, in response to PBR ligand activation, was identical (0.9 nmol/mg protein/min) for adrenal (Krueger, K.E. and Papadopoulos, V. 1990, *J. Biol. Chem.* 265:15015-15022) and liver (Tsankova *et al.* 1995, *supra*) mitochondria, suggesting that a similar PBR-mediated cholesterol transport mechanism is operative in both steroidogenic and non-steroidogenic tissues.

As noted above a bacterium is a model system without endogenous cholesterol (Moat and Foster, 1995, *supra*). In addition, bacteria do not express PBR protein (Fig. 2A) and ligand binding (not shown) although the presence of a PBR homologous protein, the tryptophan-rich-sensory-protein *tsp0* (also called *crtK),* involved in carotenoid biosynthesis in *Rhodobacter capsulatus* and *Rhodobacter sphaeroides* photosynthetic bacteria, has been reported (Yeliseev, A.A. and Kaplan S. 1995, *J. Biol. Chem.* 270:21167-21175). *Escherichia coli* were transfected with mouse PBR cDNA in a pET vector. Addition of IPTG to transfected bacteria resulted in the expression of the 18 kDa PBR protein (Fig. 2A) and ligand binding (Fig. 2B; Kd=1.1 nM and Bmax=0.23 pmol/mg protein) with similar pharmacological characteristics to that previously described for PBR (Papadopoulos, 1993, *supra*; Papadopoulos, V. *et al.* 1990, *supra)* (Fig. 2C). IPTG-induced PBR expression resulted in a protein (Fig. 3A), time (Fig. 3B), and temperature-dependent uptake of radiolabeled cholesterol (Fig. 3B). This cholesterol uptake was maintained when protoplasts were prepared indicating that PBR resides in the internal bacterial plasma membrane (data not shown). The uptake of cholesterol could not be blocked by energy poisons (DeGrella, R. F. and Simoni, R. d., 1982, *J. Biol. Chem.* 257: 14256-14262) (Fig. 3C). In addition, it was specific for cholesterol since no uptake of other radiolabeled steroid could be seen (Fig. 3D), and could not be saturated at the concentrations of radiolabeled cholesterol used (Fig. 3E), suggesting that PBR functions as a channel for cholesterol rather than a cholesterol-binding protein, which is in agreement with the modeling studies (Bernassau, *et al*., 1993, *supra*; Papadopoulos, V. 1996, *supra*). When IPTG-induced, cholesterol-loaded, bacterial membranes where treated with PK 11195, cholesterol was liberated from the membranes (Fig. 3F), suggesting that cholesterol captured by PBR is released upon ligand binding. Thus, PBR serves a channel-like or a port function where cholesterol can enter and reside stored within the membrane without interacting with the lipid or protein components of the lipid bilayer. This may also be the way by which the mitochondria sort between the steroidogenic pool of cholesterol from the cholesterol component of the membrane. Thus, PBR ligands control the opening/release state of the channel, mediating cholesterol movement across membranes. In addition to the PBR drug ligands, the polypeptide diazepam binding inhibitor (DBI) and porphyrins (Papadopoulos, V. 1993, *supra)* have been identified as naturally occurring endogenous ligands. It should be noted that at present we couldn't exclude the possibility that PBR functions as a flippase or a transporter.

In support of the data presented herein, targeted disruption of the PBR gene in steroidogenic cells resulted in inhibition of cholesterol transport to IMM and arrest of steroid biosynthesis (Papadopoulos, V. *et al.* 1997, *J. Biol. Chem.* 272:32129-32135). Transfecting PBR mutant Leydig cells with the PBR cDNA rescued steroidogenesis and demonstrated the obligatory role of PBR in cholesterol transport (Papadopoulos, V. 1996, *supra).*

### Example 2

PBR is an 18 kDa hydrophobic protein with five putative transmembrane domains located in the OMM. As a first step in defining the regions of the receptor involved in the interaction with the drug ligand and cholesterol, we constructed mutant PBRs with the deletions indicated in the left panel of Fig. 4. The location of the five transmembrane regions of the receptor (I to V) is also shown in Fig. 4. It should be noted that the amino-terminus of mitochondrial membrane proteins is directed towards the inside of the organelle whereas the carboxy-terminus is in the cytoplasmic side. The right panel of Fig. 4 shows the effect of deletion of specific amino acid sequences on PBR ligand binding and cholesterol uptake examined in the bacterial system described above. Deletion of amino acid sequences 5-20 and 41-51 in the amino-terminus of the receptor decreased by 30-45% the ability of PBR to bind the ligand PK 11195. Our results are in agreement with previous studies on human PBR expressed in yeast (Farges, R. *et al.,* 1994, *Mol. Pharm.* 46:1160-1167), although in those studies deletion of the amino-terminus of human PBR completely abolished the ability of the receptor to bind PK 11195. Deletion of amino acids 120-133 in the fourth transmembrane domain also decreased PBR ligand binding by 45%. Smaller decrease (25%) of PK 11195 binding was also seen when the regions 141-152 and 153-169 were deleted. These results suggest that although the amino-terminus of the receptor may confer the ability to bind drug ligands, such as the isoquinoline PK 11195, amino acid sequences in the fourth transmembrane domain may participate in the formation of the ligand binding site. It should be noted that deletions affecting PK 11195 ligand binding did not had a major effect on the ability of the recombinant receptor expressed in bacterial membranes to take up radiolabeled cholesterol.

Fig. 4 also shows that deletion of amino acids 153-169 in the carboxy-terminus of PBR had a dramatic effect on the ability of the molecule to take up ³H-cholesterol when expressed in bacteria (70% decrease). This result suggests that the cytoplasmic carboxy-terminal domain of the receptor is responsible for the interaction and subsequent uptake of cholesterol. In an effort to identify specific amino acids in PBR responsible for the interaction with cholesterol we undertook site-directed mutagenesis studies in the carboxy-terminal region.

### Example 3

Recent studies by Pikuleva *et al.* (1995, *Arch. Biochem. Biophys.* 322:189-197) with another protein that interacts with cholesterol, the enzyme P450scc, indicated that a Tyrosine in the active site of the P450scc interacts with the side chain of cholesterol. Aligning the P450scc active site amino acid sequence with the carboxy-terminus of PBR indicated that there might be a common amino acid consensus pattern in these two molecules recognizing cholesterol (Table I). This consensus pattern is composed of a neutral and hydrophobic amino acid (Z), such as Leucine or Valine, a neutral and polar amino acid (Y), such as Tyrosine, and a basic amino acid (Q), such as Arginine or Lysine. One to five different amino acids may be placed between these three coding amino acids. Thus, the proposed consensus pattern is **-Z-(X)**_{**0-5**}**-Y-(X)**_{**0-5-**}**Q-.** Leucine or Valine will interact with the hydrophobic side chain of cholesterol and Tyrosine will interact with the polar 3'OH-group of cholesterol, whereas the Arginine or Lysine may help create a pocket. This hypothesis was tested (Fig. 5). Replacement of Y153 by Serine or R156 by Leucine completely abolished the ability of PBR to take up radiolabeled cholesterol. Mutation and replacement of A147 with Threonine, did not affect cholesterol uptake by bacteria expressing the mutated receptor. Fig. 5 also shows that the wild-type and mutated recombinant receptor proteins were expressed at equal levels upon IPTG induction.

### Example 4

In an effort to see whether this putative cholesterol recognition/interaction amino acid consensus pattern is present in other molecules shown or suggested to interact with cholesterol, such as the as apolipoprotein A-1 (Boyle, T. P.and Marotti, K.R., 1992, *Gene* 117, 243-247), caveolin (Murata, M. *et al.* 1995, *Proc. Natl. Aca. Aci. USA* 92, 10339-10343), DBI (Papadopoulos, V. 1993, *Endocr. Rev.* 14, 222-240; Papadopoulos, V. 1998, *Proc. Soc. Exp. Biol. Med.* 217, 130-142), steroidogenesis acute regulatory protein (StAR) (Stocco, D. M. and Clark, B. J. 1996, *Endocr. Rev.* 17, 221-244), hedgehog protein (Porter, J. A. *et al.* 1996, *Science* 274, 255-259), cytochrome P450 C26/25 (Su, P. *et al.* 1990, *DNA Cell Biol.* 9-657-667), annexin II (Harder, T. *et al.* 1997, *Mol. Biol. Cell 8,* 533-545), sterol carrier protein-2 (Colles, *S. M. et al.* 1995, *Lipids* 30, 795-803), cholesterol 7α-monooxygenase (Kai, M. *et al.* 1995, *Lipid Res.* 36, 367-374), cholesterol oxidase (Ishizaki, T. *et al.* 1991, *J. Bacteriol.* 171, 596-601), cholesterol dehydrogenase (Horinouchi, S. *et al.* 1991, *Appl. Environ. Microbiol.* 57, 1386-1393), bile-salt-activated lipase precursor (cholesterol esterase) (Nilsson, *J. et al.* 1990, *Eur. J. Biochem.* 192, 543-550), and acyl-CoA cholesterol acyltransferase (Pape, M.E. *et al.* 1995, *J. Lipid Res.* 36, 823-838) we looked for the presence of the cholesterol recognition/interaction amino acid consensus pattern - **Z-(X)**_{**0-5**}**-Y-(X)**_{**0-5**}**-Q-** in these proteins. Table I shows that all these proteins, with the exception of sterol carrier protein-2, contain this amino acid consensus pattern. Proteins such as rat skeletal muscle alpha-actin, non-muscle and smooth muscle myosin light chain did not contain this cholesterol recognition/interaction consensus pattern. However, given any tyrosine there is a reasonably high probability that this consensus amino acid sequence will be found in many proteins. Indeed, a motif search through the various gene data banks indicated that this amino acid consensus pattern is present in various proteins. This is not surprising since it'is known that the cholesterol/protein interaction plays a role not only in cholesterol transport and/or storage but also in protein stability, folding, and/or localization. Thus, it is possible that only in some proteins this consensus sequence will be functional. The strength and specificity of the interaction of a protein containing this consensus amino acid sequence with cholesterol may be due either to the presence of a certain microenvironment, or the location of the consensus sequence within the protein, or a specific conformation of the protein that allows the use of this amino acid sequence. In the latter case, it is also possible that the consensus sequence identified represents only a portion, maybe the core, of a larger motif to be identified.

Of special interest to steroidogenesis is the observation that the cholesterol recognition/interaction amino acid consensus pattern was found in the polypeptide DBI (Papadopoulos, V. 1993, *supra*; Papadopoulos, V. 1998, *supra)* and the precursor StAR protein (Stocco, D. M. and Clark, B. J. 1996, *Endocr. Rev.* 17, 221-244). In search of a cytosolic steroidogenesis-stimulating factor, a protein was purified shown to be identical to DBI (Papadopoulos, V. 1993, *supra).* DBI was originally purified from brain by monitoring its ability to displace diazepam from its recognition sites in synaptosomes. DBI is also identical to the acyl-CoA-binding protein (Knudsen, J. *et al.,* 1993, *Mol. Cell. Biochem.* 123:129-138). Purified DBI was shown to stimulate intramitochondrial cholesterol transport and increase pregnenolone formation by isolated mitochondria (Papadopoulos, V., 1998, *supra*). Later on, it was demonstrated that this action of DBI was mediated by PBR (Papadopoulos, V. 1993, *supra;* Papadopoulos, V. 1998, *supra).* In addition DBI was shown to increase cholesterol loading onto isolated P450scc (Brown, A. S. and Hall, P. F., 1991 *Biochem. Biophys. Res. Commun.* 180:609-614). Thus, the identification of the cholesterol recognition/interaction amino acid consensus pattern in DBI may help understand its role in steroidogenesis and its direct effect on P450scc. Interestingly, we showed in the past that the naturally occurring processing product of DBI, the triakontatetrapeptide TTN (DBI₁₇₋₅₀), but not the octadecaneuropeptide ODN (DBI₃₃₋₅₀), was able to mimic the effect of DBI on mitochondrial steroidogenesis (Papadopoulos, V. *et al.* 1991, *Endocrinology* 129:1481-1488). The finding that in DBI the cholesterol recognition/interaction amino acid consensus pattern is located in the amino acid sequence 25 to 32 (Table I) may now explain this result. It should be also noted that the cholesterol recognition/interaction amino acid consensus pattern is found in the middle of the acyl-CoA-binding protein signature domain (amino acids 19 to 37) important in forming the acyl-CoA-binding site (Knudsen *et al.* 1993, *supra*).

StAR has been found in gonadal and adrenal cells, where it is newly synthesized in response to trophic hormones, as a cytoplasmic precursor protein of 37 kDa targeted to mitochondria (Stocco and Clark, 1996 *supra*). StAR synthesis in Leydig cells begins 60 min after addition of the hormone and then parallels the capacity of the cells to produce steroids in response to tropic hormones (Stocco and Clark, 1996 *supra;* Clark, B. J. *et al.,* 1995, *Mol. Endocr.* 9:1346-1355). The 37 kDa StAR precursor further undergoes cleavage to produce the 30 kDa mitochoddrial "mature" StAR protein and its phosphorylated counterpart (Stocco and Clark, 1996 *supra*). This protein processing is believed to occur at the level of the outer/inner mitochondrial membrane contact sites and it has been proposed to be responsible for cholesterol transport from outer to inner mitochondrial membrane (Stocco and Clark, 1996 *supra*). Considering that the cholesterol recognition/interaction amino acid consensus pattern is found in the amino-terminus of the StAR precursor protein, which is removed from the mature protein, it is possible that the function of the precursor StAR protein is to shuttle cholesterol from intracellular stores to the outer mitochondrial membrane.

In conclusion, the results presented herein demonstrate that PBR may have a channel-like function for cholesterol in the OMM. The steroidogenic pool of cholesterol, coming from various intracellular sources, is recognized by the cholesterol recognition/interaction amino acid consensus pattern - **Z-(X)**_{**0-5**}**-Y-(X)**_{**0-5**}**-Q-** present in the carboxy-terminus of PBR in the OMM. This pool of cholesterol enters in the OMM at the PBR sites where it remains without mixing with other membrane components. Ligand binding to the receptor induces the release of this cholesterol. Considering that PBR has been shown to be associated with the voltage-dependent anion channel (Papadopoulos, V. 1998, *supra),* found in the outer/inner mitochondrial membrane contact sites, the released cholesterol could now directly access the P450scc in the IMM where it will be cleaved to pregnenolone, precursor of all steroids.

In addition to being a precursor for steroid hormone synthesis, cholesterol is an essential structural element of cellular membranes and a precursor for the synthesis of bile acids and lipoproteins. Mammalian cells obtain cholesterol by internalization of low-density lipoproteins or by *de novo* synthesis in the endoplasmic reticulum. The subcellular distribution of cholesterol suggests that cholesterol is trafficked and incorporated quickly from the sites of acquisition to the target membrane (Liscum, L and Underwood, K. W. 1995, *J. Biol. Chem.* 270:15433-15446). Thus, a tissue and cell specific cholesterol homeostasis is achieved. Considering the widespread occurrence of PBR and its tissue and cell specific subcellular localization (Papadopoulos, V. 1993, *supra;* Papadopoulos, V. 1998, *supra),* these results suggest a more general role for PBR in intracellular cholesterol transport and compartmentalization.

## Claims

1. An isolated peptide having a cholesterol recognition/interaction amino acid sequence selected from the group consisting of SEQ ID NO:2, SEQ ID NO:3, SEQ ID NO:4 and SEQ ID NO:5.

2. An isolated nucleic acid molecule comprising a cholesterol interaction/recognition nucleotide sequence from the group consisting of:
a) a nucleotide sequence encoding a cholesterol recognition/interaction amino acid sequence as defined in claim 1;
b) a nucleotide sequence complementary to (a).

3. A nucleic acid molecule comprising:
(i) a vector; and
(ii) a nucleic acid molecule as defined in claim 2.

4. The nucleic acid molecule according to claim 3, wherein said vector is a prokaryotic vector.

5. The nucleic acid molecule according to claim 3, wherein said vector is a eukaryotic vector.

6. The nucleic acid molecule according to claim 4 or claim 5, wherein said vector is an expression vector.

7. The nucleic acid molecule according to claim 6, wherein said vector is useful for expression in plants.

8. A host cell transformed with the nucleic acid molecule of claim 3.

9. The host cell of claim 8, wherein said host cell is prokaryotic.

10. The host cell of claim 8, wherein said host cell is eukaryotic.

11. The host cell of claim 10, wherein said host is a plant cell.

12. A method for detecting whether a protein recognizes cholesterol comprising identifying in the amino acid sequence of said protein, the presence or absence of a cholesterol interaction/recognition amino acid sequence as defined in claim 1, or identifying in the nucleotide sequence encoding said protein, the presence or absence of a cholesterol interaction/recognition nucleotide sequence as defined in claim 2, wherein the presence of a cholesterol recognition/interaction amino acid or the presence of a cholesterol recognition/interaction nucleotide sequence indicates interaction/recognition of the protein with cholesterol.

13. A method for conferring cholesterol recognition/interaction to a molecule comprising introducing into said molecule a cholesterol recognition/interaction amino acid sequence as defined in claim 1 such that said sequence is expressed and said molecule interacts with cholesterol.

14. Use of a peptide according to claim 1 for the manufacture of a medicament for regulating the amount of cholesterol in the blood or tissue.

15. Use according to claim 14, wherein the medicament for regulating the amount of cholesterol in the blood is used for the treatment of gallstones, atherosclerosis, Niemann-Pick C, Sitosterolemia, Dystrophy, tumorigenesis, Schnyder's corneal crystalline dystrophy, Alzheimer's disease, Tellurium toxicity, Smith-Lemli-Opitz syndrome, myelinization, Charcot-Marie-Tooth disease, Pelizaeus-Merzbacher disease and multiple sclerosis.

16. Use of a nucleic acid according to claim 2 for the preparation of a composition for reducing the serum cholesterol level in a subject.

17. Use of a peptide comprising the cholesterol interaction/recognition amino acid sequence as defined in claim 1 complexed with a pharmaceutically acceptable amount of cholesterol and a pharmaceutically acceptable diluent in the preparation of a composition for delivering cholesterol to a subject.

18. A method for detecting an increase or decrease of cholesterol in a biological sample comprising:
immobilizing a polypeptide comprising the cholesterol interaction/recognition amino acid sequence as defined in claim 1 on a solid support rendering an immobilized polypeptide
exposing the sample to the immobilized polypeptide, and
measuring the amount of cholesterol bound polypeptide wherein when comparing to a standard an increase or decrease over the standard can be determined.

19. A method for screening agents or drugs which are agonists or antagonists of interaction between peptides comprising the cholesterol interaction/recognition amino acid sequence as defined in claim 1 and cholesterol comprising:
exposing a peptide comprising the cholesterol interaction/recognition amino acid sequence as defined in claim 1 to cholesterol under conditions where interaction between cholesterol and the peptide occurs forming a peptide/cholesterol complex
incubating the complex with a test compound
measuring an increase or decrease in the level of interaction between the peptide and cholesterol in response to the test compound where an increase in interaction would indicate that the test compound is an agonist and a decrease in interaction would indicate that the test compound is an antagonist of peptide/cholesterol binding.

20. A method according to claim 19, which is for screening for antagonists of interaction between peptides comprising the cholesterol interaction/recognition amino acid sequence as defined in claim 1 and cholesterol, wherein the test compound is a peptide, drug or antibody.

21. A method according to claim 20, which method further comprises a step of producing an antagonist identified in the method.

22. A method for reducing the cholesterol binding ability of a peptide which comprises the cholesterol recognition/interaction amino acid sequence as defined in claim 1 comprising:
modifying a tyrosine to a serine,
modifying an arginine to a leucine,
modifying a threonine to a serine,
modifying a valine to a leucine,
modifying a leucine to isoleucine,
modifying a tyrosine to a valine,
modifying a tyrosine to a isoleucine,
modifying a valine to a isoleucine,
modifying a arginine to a isoleucine, and
modifying a aspartate to a glutamate.

23. A peripheral-type benzodiazepine receptor wherein the cholesterol recognition/interaction function of said receptor is reduced according to the method of claim 22.

24. A peripheral-type benzodiazepine receptor unable to recognize/interact with cholesterol, which receptor has a deletion comprising a cholesterol interaction/recognition amino acid sequence as defined in claim 1.

25. Use of a nucleic acid as defined in claim 2 for the preparation of a composition for reducing disease symptoms in a subject resulting from an increase in cholesterol.

26. The use according to claim 25, wherein said composition comprises microspheres.

27. A transgenic plant comprising a nucleic acid encoding a peripheral-type benzodiazepine receptor according to claim 24 or claim 25.

28. A transgenic plant comprising a nucleic acid encoding a peripheral-type benzodiazepine receptor according to claim 24 or claim 25 operably linked to an inducible promoter.

29. The transgenic plant according to claim 28, wherein said promoter is inducible in any of the following conditions: heat, administration of antibiotic, administration of plant hormone.

## Patentansprüche

1. Ein isoliertes Peptid, das eine Aminosäuresequenz zur Cholesterinerkennung/-wechselwirkung aufweist, die aus der Gruppe ausgewählt ist, die aus SEQ ID Nr. 2, SEQ ID Nr. 3, SEQ ID Nr. 4 und SEQ ID Nr. 5 besteht.

2. Ein isoliertes Nukleinsäuremolekül, das eine Nukleotidsequenz zur Cholesterinwechselwirkung/-erkennung aus der Gruppe enthält, die aus
a) einer Nukleotidsequenz, die eine wie in Anspruch 1 definierte Aminosäuresequenz zur Cholesterinerkennung/-wechselwirkung kodiert,
b) einer zu a) komplementären Nukleotidsequenz
besteht.

3. Ein Nukleinsäuremolekül, enthaltend:
(i) einen Vektor und
(ii) ein wie in Anspruch 2 definiertes Nukleinsäuremolekül.

4. Das Nukleinsäuremolekül nach Anspruch 3, wobei der Vektor ein prokariotischer Vektor ist.

5. Das Nukleinsäuremolekül nach Anspruch 3, wobei der Vektor ein eukariotischer Vektor ist.

6. Das Nukleinsäuremolekül nach Anspruch 4 oder Anspruch 5, wobei der Vektor ein Expressionsvektor ist.

7. Das Nukleinsäuremolekül nach Anspruch 6, wobei der Vektor zur Expression in Pflanzen verwendbar ist.

8. Eine mit dem Nukleinsäuremolekül des Anspruchs 3 transformierte Wirtszelle.

9. Die Wirtszelle des Anspruchs 8, wobei die Wirtszelle prokariotisch ist.

10. Die Wirtszelle des Anspruchs 8, wobei die Wirtszelle eukariotisch ist.

11. Die Wirtszelle des Anspruchs 10, wobei die Wirtszelle eine Pflanzenzelle ist.

12. Ein Verfahren zur Bestimmung, ob ein Protein Cholesterin erkennt, enthaltend: Feststellen der Anwesenheit oder Abwesenheit einer wie in Anspruch 1 definierten Aminosäuresequenz zur Cholesterinwechselwirkung/-erkennung in der Aminosäuresequenz des Proteins oder Feststellen der Anwesenheit oder Abwesenheit einer wie in Anspruch 2 definierten Nukleotidsequenz zur Cholesterinwechselwirkung/-erkennung in der Nukleotidsequenz, die das Protein kodiert, wobei die Anwesenheit einer Aminosäuresequenz zur Cholesterinerkennung/-wechselwirkung oder die Anwesenheit einer Nukleotidsequenz zur Cholesterinerkennung/-wechselwirkung die Wechselwirkung/Erkennung des Proteins mit/von Cholesterin anzeigt.

13. Ein Verfahren zur Übertragung von Cholesterinerkennung/-wechselwirkung auf ein Molekül, enthaltend: Einführen einer wie in Anspruch 1 definierten Aminosäuresequenz zur Cholesterinerkennung/-wechselwirkung in das Molekül, so dass die Sequenz exprimiert wird und das Molekül mit Cholesterin wechselwirkt.

14. Verwendung eines Peptids nach Anspruch 1 zur Herstellung eines Medikaments zur Regulierung der Cholesterinmenge in Blut oder Gewebe.

15. Verwendung nach Anspruch 14, wobei das Medikament zur Regulierung der Cholesterinmenge im Blut zur Behandlung von Gallensteinen, Atherosklerose, Niemann-Pick C, Sitosterolemie, Dystrophie, Tumorgenese, Schnyderscher kristalliner Hornhautdystrophie, Alzheimer-Krankheit, Tellurvergiftung, Smith-Lemli-Opitz-Syndrom, Myelinisation, Charcot-Marie-Tooth-Krankheit, Pelizaeus-Merzbacher-Krankheit und Multipler Sklerose verwendet wird.

16. Verwendung einer Nukleinsäure nach Anspruch 2 zur Herstellung einer Zusammenstellung zur Reduzierung des Serum-Cholesterinspiegels in einem Subjekt.

17. Verwendung eines Peptids, das die wie in Anspruch 1 definierte Aminosäuresequenz zur Cholesterinwechselwirkung/-erkennung enthält, welches mit einer pharmazeutisch geeigneten Menge an Cholesterin komplexiert ist und eines pharmazeutisch geeigneten Lösungsmittels in der Herstellung einer Zusammenstellung zur Verabreichung von Cholesterin an ein Subjekt.

18. Ein Verfahren zur Feststellung eines Anstiegs oder Abfalls von Cholesterin in einer biologischen Probe, enthaltend:
Immobilisieren eines Polypeptids, das die wie in Anspruch 1 definierte Aminosäuresequenz zur Cholesterinwechselwirkung/-erkennung enthält, auf einem festen Träger, um ein immobilisiertes Polypeptid zu erhalten,
Behandeln der Probe mit dem immobilizierten Polypeptid und
Messen der Polypeptidmenge, die an Cholesterin gebunden ist, wobei ein Anstieg oder Abfall bezüglich eines Standards bestimmt werden kann, wenn mit einem Standard verglichen wird.

19. Ein Verfahren zum Screenen von Agenzien oder Wirkstoffen, welche Agonisten oder Antagonisten der Wechselwirkung zwischen Peptiden, die die wie in Anspruch 1 definierte Aminosäuresequenz zur Cholesterinwechselwirkung/-erkennung enthalten, und Cholesterin sind, enthaltend:
Behandeln eines Peptides, das die wie in Anspruch 1 definierte Aminosäuresequenz zur Cholesterinwechselwirkung/-erkennung enthält, mit Cholesterin, unter Bedingungen, bei denen Wechselwirkung zwischen Cholesterin und dem Peptid auftritt, wobei ein Peptid-Cholesterin-Komplex gebildet wird,
Inkubieren des Komplexes mit einer Testverbindung,
Messen eines Anstiegs oder Abfalls des Wechselwirkungsgrades zwischen dem Peptid und Cholesterin in Antwort auf die Testverbindung, wobei ein Anstieg der Wechselwirkung anzeigt, dass die Testverbindung ein Agonist ist, und ein Abfall der Wechselwirkung anzeigt, dass die Testverbindung ein Antagonist für die Peptid-Cholesterinbindung ist.

20. Ein Verfahren nach Anspruch 19, welches zum Screenen von Antagonisten der Wechselwirkung zwischen Peptiden, die die wie in Anspruch 1 definierte Aminosäuresequenz zur Cholesterinwechselwirkung/-erkennung enthalten, und Cholesterin vorgesehen ist, wobei die Testverbindung ein Peptid, ein Wirkstoff oder ein Antikörper ist.

21. Ein Verfahren nach Anspruch 20, welches weiter einen Schritt zur Herstellung eines Antagonisten, der durch die Methode identifiziert wurde, enthält.

22. Ein Verfahren zur Reduzierung der Fähigkeit zur Cholesterinbindung eines Peptides, welches die wie in Anspruch 1 definierte Aminosäuresequenz zur Cholesterinerkennung/-wechselwirkung enthält, enthaltend:
Modifizieren eines Tyrosins zu einem Serin,
Modifizieren eines Arginins zu einem Leucin,
Modifizieren eines Threonins zu einem Serin,
Modifizieren eines Valins zu einem Leucin,
Modifizieren eines Leucins zu einem Isoleucin,
Modifizieren eines Tyrosins zu einem Valin,
Modifizieren eines Tyrosins zu einem Isoleucin,
Modifizieren eines Valins zu einem Isoleucin,
Modifizieren eines Arginins zu einem Isoleucin und
Modifizieren eines Aspartats zu einem Glutamat.

23. Ein Benzodiazepin-Rezeptor vom periphären Typ, wobei die Cholesterinwechselwirkungs-/-erkennungsfunktion des Rezeptors nach der Methode des Anspruchs 22 reduziert wird.

24. Ein Benzodiazepin-Rezeptor vom periphären Typ, der unfähig ist zur Cholesterinerkennung/-wechselwirkung, welcher eine Deletion, die eine wie in Anspruch 1 definierte Aminosäuresequenz zur Cholesterinwechselwirkung/-erkennung enthält, aufweist.

25. Verwendung einer wie in Anspruch 2 definierten Nukleinsäure zur Herstellung einer Zusammenstellung zur Reduzierung von Krankheitssymptomen in einem Subjekt, die aus einem Cholesterinanstieg resultieren.

26. Die Verwendung nach Anspruch 25, wobei die Zusammenstellung Mikrokugeln enthält.

27. Eine transgene Pflanze, die eine Nukleinsäure enthält, die einen Benzodiazepin-Rezeptor vom periphären Typ nach Anspruch 24 oder Anspruch 25 kodiert.

28. Eine transgene Pflanze, die eine Nukleinsäure enthält, die einen Benzodiazepin-Rezeptor vom periphären Typ gemäß Anspruch 24 oder Anspruch 25 kodiert, welche funktionsgerecht mit einen induzierbaren Promotor verknüpft ist.

29. Die transgene Pflanze nach Anspruch 28, wobei der Promotor durch eine der folgenden Bedingungen induzierbar ist: Hitze, Verabreichung eines Antibiotikums, Verabreichung eines Pflanzenhormons.

## Revendications

1. Peptide isolé ayant une séquence d'acides aminés de reconnaissance du/interaction avec le cholestérol choisie dans le groupe constitué de SEQ ID NO : 2, SEQ ID NO : 3, SEQ ID NO : 4 et SEQ ID NO : 5.

2. Molécule d'acide nucléique isolée comprenant une séquence nucléotidique d'interaction avec le/reconnaissance du cholestérol dans le groupe constitué de :
a) une séquence nucléotidique codant une séquence d'acides aminés de reconnaissance du/interaction avec le cholestérol telle que définie dans la revendication 1 ;
b) une séquence nucléotidique complémentaire à (a).

3. Molécule d'acide nucléique comprenant :
(i) un vecteur ; et
(ii) une molécule d'acide nucléique telle que définie dans la revendication 2.

4. Molécule d'acide nucléique selon la revendication 3, dans laquelle ledit vecteur est un vecteur procaryote.

5. Molécule d'acide nucléique selon la revendication 3, dans laquelle ledit vecteur est un vecteur eucaryote.

6. Molécule d'acide nucléique selon la revendication 4 ou la revendication 5, dans laquelle ledit vecteur est un vecteur d'expression.

7. Molécule d'acide nucléique selon la revendication 6, dans laquelle ledit vecteur est utile pour une expression dans des plantes.

8. Cellule hôte transformée avec la molécule d'acide nucléique selon la revendication 3.

9. Cellule hôte selon la revendication 8, pour laquelle ladite cellule hôte est procaryote.

10. Cellule hôte selon la revendication 8, pour laquelle ladite cellule hôte est eucaryote.

11. Cellule hôte selon la revendication 10, pour laquelle ledit hôte est une cellule végétale.

12. Procédé pour détecter si une protéine reconnaît le cholestérol, comprenant l'identification dans la séquence d'acides aminés de ladite protéine de la présence ou de l'absence d'une séquence d'acides aminés d'interaction avec le/reconnaissance du cholestérol telle que définie dans la revendication 1, ou l'identification dans la séquence nucléotidique codant ladite protéine de la présence ou de l'absence d'une séquence nucléotidique d'interaction avec le/reconnaissance du cholestérol telle que définie dans la revendication 2, dans lequel la présence d'une séquence d'acides aminés de reconnaissance du/d'interaction avec le cholestérol ou la présence d'une séquence nucléotidique de reconnaissance du/interaction avec le cholestérol indique une interaction/une reconnaissance de la protéine avec le cholestérol.

13. Procédé pour conférer à une molécule une reconnaissance du/une interaction avec le cholestérol comprenant l'introduction dans ladite molécule d'une séquence d'acides aminés de reconnaissance du/interaction avec le cholestérol telle que définie dans la revendication 1 de sorte que ladite séquence soit exprimée et que ladite molécule interagisse avec le cholestérol.

14. Utilisation d'un peptide selon la revendication 1 pour la fabrication d'un médicament pour réguler la quantité de cholestérol dans le sang ou un tissu.

15. Utilisation selon la revendication 14, dans laquelle le médicament pour réguler la quantité de cholestérol dans le sang est utilisé pour le traitement de calculs biliaires, d'une athérosclérose, d'une maladie de Niemann-Pick C, d'une sitostérolémie, d'une dystrophie, d'une tumorigenèse, d'une dystrophie cristalline de la cornée de Schnyder, d'une maladie de Alzheimer, d'une toxicité du tellurium, d'un syndrome de Smith-Lemli-Opitz, d'une myélinisation, d'une amyotrophie péronière de Charcot-Marie-Tooth-Hoffmann, d'une maladie de Pelizaeus-Merzbacher et d'une sclérose en plaques.

16. Utilisation d'un acide nucléique selon la revendication 2 pour la préparation d'une composition pour la réduction du niveau de cholestérol dans le sérum chez un sujet.

17. Utilisation d'un peptide comprenant la séquence d'acides aminés d'interaction avec le/reconnaissance du cholestérol telle que définie dans la revendication 1 complexée avec une quantité acceptable d'un point de vue pharmaceutique de cholestérol et un diluant acceptable d'un point de vue pharmaceutique dans la préparation d'une composition pour délivrer du cholestérol à un sujet.

18. Procédé de détection d'une augmentation ou d'une réduction du cholestérol dans un échantillon biologique comprenant :
l'immobilisation d'un polypeptide comprenant la séquence d'acides aminés d'interaction avec le/reconnaissance du cholestérol telle que définie dans la revendication 1 sur un support solide, rendant un polypeptide immobilisé
l'exposition de l'échantillon au polypeptide immobilisé, et
la mesure de la quantité de polypeptide lié au cholestérol, dans lequel, lors de la comparaison avec un standard, on peut déterminer une augmentation ou une réduction par rapport au standard.

19. Procédé de criblage d'agents ou de médicaments qui sont des agonistes ou des antagonistes d'une interaction entre des peptides comprenant la séquence d'acides aminés d'interaction avec le/reconnaissance du cholestérol telle que définie dans la revendication 1 et le cholestérol comprenant :
l'exposition d'un peptide comprenant la séquence d'acides aminés d'interaction avec le/reconnaissance du cholestérol telle que définie dans la revendication 1 au cholestérol dans des conditions dans lesquelles l'interaction entre le cholestérol et le peptide intervient, formant un complexe peptide/cholestérol,
l'incubation du complexe avec un composé test
la mesure d'une augmentation ou d'une réduction du niveau d'interaction entre le peptide et le cholestérol en réponse au composé test où une augmentation de l'interaction indiquerait que le composé test est un agoniste et une réduction de l'interaction indiquerait que le composé test est un antagoniste de la liaison peptide/cholestérol.

20. Procédé selon la revendication 19, qui est destiné au criblage d'antagonistes d'une interaction entre des peptides comprenant la séquence d'acides aminés d'interaction avec le/reconnaissance du cholestérol telle que définie dans la revendication 1 et le cholestérol, dans lequel le composé test est un peptide, un médicament ou un anticorps.

21. Procédé selon la revendication 20, lequel procédé comprend en outre une étape de production d'un antagoniste identifié dans le procédé.

22. Procédé de réduction de la capacité de liaison au cholestérol d'un peptide qui comprend la séquence d'acides aminés de reconnaissance du/interaction avec le cholestérol telle que définie dans la revendication 1 comprenant :
la modification d'une tyrosine en une sérine,
la modification d'une arginine en une leucine,
la modification d'une thréonine en une sérine,
la modification d'une valine en une leucine,
la modification d'une leucine en une isoleucine,
la modification d'une tyrosine en une valine,
la modification d'une tyrosine en une isoleucine,
la modification d'une valine en une isoleucine,
la modification d'une arginine en une isoleucine et
la modification d'un aspartate en un glutamate.

23. Récepteur de benzodiazépine de type périphérique dans lequel la fonction de reconnaissance du/interaction avec le cholestérol dudit récepteur est réduite selon le procédé selon la revendication 22.

24. Récepteur de benzodiazépine de type périphérique incapable de reconnaître/d'interagir avec le cholestérol, lequel récepteur a une délétion comprenant une séquence d'acides aminés d'interaction avec le/reconnaissance du cholestérol telle que définie dans la revendication 1.

25. Utilisation d'un acide nucléique tel que défini dans la revendication 2 pour la préparation d'une composition pour réduire les symptômes pathologiques chez un sujet résultant d'une augmentation du cholestérol.

26. Utilisation selon la revendication 25, dans lequel ladite composition comprend des microsphères.

27. Plante transgénique comprenant un acide nucléique codant un récepteur de benzodiazépine de type périphérique selon la revendication 24 ou 25.

28. Plante transgénique comprenant un acide nucléique codant un récepteur de benzodiazépine de type périphérique selon la revendication 24 ou la revendication 25, lié de manière fonctionnelle à un promoteur inductible.

29. Plante transgénique selon la revendication 28, dans laquelle ledit promoteur est inductible dans l'une quelconque des conditions suivantes : chaleur, administration d'un antibiotique, administration d'une hormone végétale.
